# EUROPEAN PATENT APPLICATION

(11) **EP 4 835 047 A1**
(43) Date of publication of application: **04.11.2026**
(21) Application number: 24911111.3
(22) Date of filing: 24.12.2024
(51) Int. Cl.: C12M 1/34, C12M 1/00, C12P 21/00

(54) **AUTOMATIC MATERIAL SUPPLEMENTING METHOD BASED ON REAL-TIME DYNAMIC MEASUREMENT RESULT**

(30) Priority: 28.12.2023 CN 202311837262
(71) Applicant: Wuxi Biologics Ireland Limited, Dundalk, Co Louth A91 X56F (IE)
(72) Inventor: TANG, Hao, Wuxi, Jiangsu 214000 (CN); XIANG, Shaoxun, Wuxi, Jiangsu 214000 (CN); TANG, Wenxiu, Wuxi, Jiangsu 214000 (CN); SU, Yuning, Wuxi, Jiangsu 214000 (CN); YU, Le, Wuxi, Jiangsu 214000 (CN)
(74) Representative: Santarelli
(86) International application number: PCT/CN2024/141814
(87) International publication number: WO 2025/140186

(57) **Abstract**

An automatic material supplementing method based on a real-time dynamic measurement result includes: collecting a real-time dynamic measurement result, and calculating a rate of change of each component within a unit time, where the rate of change is a consumption rate or a generation rate; selecting a component having the highest rate of change as a marker for a current time period; collecting historical measurement data, and calculating a cell growth rate in a logarithmic growth phase and a target product expression rate in a culture harvest phase; taking, as a component concentration set value for the current time period of the logarithmic growth phase, a concentration level of the marker when the cell growth rate in the logarithmic growth phase is highest; taking, as a component concentration set value for the current time period of the culture harvest phase, a concentration level of the marker when the target product expression rate in the culture harvest phase is highest; and calculating an addition amount of the marker for the current time period based on the rate of change of the marker and/or based on a difference between a real-time measured concentration of the marker for the current time period and the component concentration set value of the marker for the current time period, and calculating an addition amount of a culture medium based on the addition amount of the marker to obtain a material supplementing strategy.

## Description

### CROSS-REFERENCE TO RELATED APPLICATIONS

This application is based on and claims priority to CN application No. 202311837262.9, filed on December 28, 2023, the disclosure of which is incorporated herein by reference in its entirety.

### TECHNICAL FIELD

The present disclosure relates to the field of biotechnologies, and specifically to an automatic material supplementing method based on a real-time dynamic measurement result.

### BACKGROUND

Currently, cell culture has been widely applied in the research, development and production of biopharmaceuticals, and a crucial factor in ensuring the success of cell culture is the cell culture process. The quality of cell culture process largely depends on the control of culture conditions in bioreactors. In addition to maintaining important physical and chemical conditions such as pH, temperature, and dissolved oxygen (DO), meeting the nutritional needs of cells plays a crucial role in establishing a suitable growth environment. The metabolism of mammalian cells involves complex metabolic pathways, in which cells consume large amounts of nutrients, such as carbon sources, nitrogen sources, and trace elements. Currently, a comprehensive supplemental culture medium is commonly used in the industry, and is formed by combining nutrients such as carbon sources, nitrogen sources, and trace elements according to the metabolic needs of cells.

### SUMMARY

A brief overview of the present disclosure is given below to provide a basic understanding of some aspects of the present disclosure. However, it should be understood that this overview is not an exhaustive overview of the present disclosure. It is not intended to identify any key or essential part of the present disclosure, nor is it intended to limit the scope of the present disclosure. Its purpose is merely to present certain concepts of the present disclosure in a simplified form as a prelude to the more detailed description that will follow.

In a first aspect, the present disclosure provides an automatic material supplementing method based on a real-time dynamic measurement result, the automatic material supplementing method including:
collecting the real-time dynamic measurement result, and calculating a rate of change of each component within a unit time, where the rate of change is a consumption rate or a generation rate; selecting a component having the highest rate of change as a marker for a current time period;
collecting historical measurement data, and calculating a cell growth rate in a logarithmic growth phase and a target product expression rate in a culture harvest phase; taking, as a component concentration set value for the current time period of the logarithmic growth phase, a concentration level of the marker when the cell growth rate in the logarithmic growth phase is highest; taking, as a component concentration set value for the current time period of the culture harvest phase, a concentration level of the marker when the target product expression rate in the culture harvest phase is highest; and
calculating an addition amount of the marker for the current time period based on the rate of change of the marker, and calculating an addition amount of a culture medium based on the addition amount of the marker to obtain a material supplementing strategy.

In some embodiments, a step of selecting the marker includes:
collecting a current real-time dynamic measurement result, and calculating a rate of change of each component within a current unit time;
collecting a historical real-time dynamic measurement result, and calculating a rate of change of each component within a historical unit time; and
combining the rate of change of each component within the current unit time with the rate of change of each component within the historical unit time, and selecting a component having the highest rate of change therefrom as the marker for the current time period.

In some embodiments, the historical measurement data is historical measurement data of a fermentation process with the same cell culture process mode as a present fermentation process.

In some embodiments, a step of calculating the addition amount of the culture medium includes:
${m}_{suppl} = \left({G}_{set}-{G}_{rt}\right) \times {V}_{cs} / {G}_{suppl} ,$
where mₛᵤₚₚₗ represents an amount of a supplemental culture medium to be added, Gₛₑₜ represents a set concentration of the marker, Gᵣₜ represents a current concentration of the marker measured by analyzing a Raman spectrum in real time with a model, V_{cs} represents a volume of a cell culture solution in a bioreactor, and Gₛᵤₚₚₗ represents a concentration of the marker in the supplemental culture medium.

In some embodiments, the supplemental culture medium includes a specific proportion of the marker and another component, and an addition amount of the other component is obtained based on the addition amount of the marker.

In some embodiments, the method further includes writing the material supplementing strategy into a control system after obtaining the material supplementing strategy.

In some embodiments, the control system is configured to perform material supplementing instantaneously in a dose-wise manner by cooperating with a peristaltic pump hardware upon receiving an numerical instruction on a supplemental amount, a set value, and a time point for material supplementing.

In a second aspect, the present disclosure provides an automatic material supplementing apparatus based on a real-time dynamic measurement result, the automatic material supplementing apparatus including:
a marker selecting unit configured to collect the real-time dynamic measurement result, and to calculate a rate of change of each component within a unit time, where the rate of change is a consumption rate or a generation rate, and to select a component having the highest rate of change as a marker for a current time period;
a marker concentration setting unit configured to collect historical measurement data and to calculate a cell growth rate in a logarithmic growth phase and a target product expression rate in a culture harvest phase, to take, as a component concentration set value for the current time period of the logarithmic growth phase, a concentration level of the marker when the cell growth rate in the logarithmic growth phase is highest, and to take, as a component concentration set value for the current time period of the culture harvest phase, a concentration level of the marker when the target product expression rate in the culture harvest phase is highest; and
a supplemental amount calculation unit configured to calculate an addition amount of the marker for the current time period based on the rate of change of the marker, and to calculate an addition amount of a culture medium based on the addition amount of the marker to obtain a material supplementing strategy.

In some embodiments, a step of selecting the marker in the marker selecting unit includes:
collecting a current real-time dynamic measurement result, and calculating a rate of change of each component within a current unit time;
collecting a historical real-time dynamic measurement result, and calculating a rate of change of each component within a historical unit time; and
combining the rate of change of each component within the current unit time with the rate of change of each component within the historical unit time, and selecting a component having the highest rate of change therefrom as the marker for the current time period.

In some embodiments, in the marker concentration setting unit, the historical measurement data is historical measurement data of a fermentation process with the same cell culture process mode as a present fermentation process.

In some embodiments, in the supplemental amount calculation unit, a step of calculating the addition amount of the culture medium includes:
${m}_{suppl} = \left({G}_{set}-{G}_{rt}\right) \times {V}_{cs} / {G}_{suppl}$
where mₛᵤₚₚₗ represents an amount of a supplemental culture medium to be added, Gₛₑₜ represents a set concentration of the marker, Gᵣₜ represents a current concentration of the marker measured by analyzing a Raman spectrum in real time with a model, V_{cs} represents a volume of a cell culture solution in a bioreactor, and Gₛᵤₚₚₗ represents a concentration of the marker in the supplemental culture medium.

In some embodiments, in the supplemental amount calculation unit, the supplemental culture medium includes a specific proportion of the marker and another component, and an addition amount of the other component is obtained based on the addition amount of the marker.

In some embodiments, in the supplemental amount calculation unit, writing the material supplementing strategy into a control system is further included after obtaining the material supplementing strategy.

In some embodiments, the control system is configured to perform material supplementing instantaneously in a dose-wise manner by cooperating with a peristaltic pump hardware upon receiving an numerical instruction on a supplemental amount, a set value, and a time point for material supplementing.

In a third aspect, the present disclosure provides an automatic material supplementing method based on a real-time dynamic measurement result, the method including:
obtaining a historical real-time dynamic measurement result, calculating a historical rate of change of each component based on the historical real-time dynamic measurement result, and selecting one component or a combination of multiple components as a marker based on the historical rate of change of each component;
collecting a current real-time dynamic measurement result, and calculating a real-time measured concentration of the marker for a current time period based on the current real-time dynamic measurement result; and
obtaining a material supplementing strategy by at least one of:
   calculating a cell growth rate in a logarithmic growth phase and a target product expression rate in a culture harvest phase based on the historical real-time dynamic measurement result, taking, as a component concentration set value of the marker for the current time period, a concentration level of the marker when the cell growth rate in the logarithmic growth phase is highest in a case that the current time period falls into the logarithmic growth phase, and taking, as a component concentration set value of the marker for the current time period, a concentration level of the marker when the target product expression rate in the culture harvest phase is highest in a case that the current time period falls into the culture harvest phase, and calculating an addition amount of a supplemental culture medium for the current time period based on a difference between the real-time measured concentration of the marker for the current time period and the component concentration set value of the marker for the current time period to obtain a first material supplementing strategy; or
   calculating a current rate of change of the marker based on the real-time measured concentration of the marker for the current time period, and calculating an addition amount of the supplemental culture medium for the current time period based on the current rate of change of the marker to obtain a second material supplementing strategy.

In some embodiments, the automatic material supplementing method further including:
calculating multiple historical rates of change of each component within multiple historical time periods based on the historical real-time dynamic measurement result; and
combining the multiple historical rates of change of each component to obtain a combined historical rate of change of each component, and selecting one component or a combination of multiple components as the marker based on the combined historical rate of change of each component.

In some embodiments, the historical real-time dynamic measurement result and the current real-time dynamic measurement result are obtained from different fermentation processes with the same cell culture process mode.

In some embodiments, calculating the addition amount of the supplemental culture medium for the current time period based on the difference between the real-time measured concentration of the marker for the current time period and the component concentration set value of the marker for the current time period includes one or more of:
in a case that the rate of change of the marker is expressed as a consumption rate,
   ${m}_{suppl} = \left({G}_{set}-{G}_{rt}\right) \times {V}_{cs} / {G}_{suppl} ,$
where mₛᵤₚₚₗ represents the addition amount of the supplemental culture medium for the current time period, Gₛₑₜ represents the component concentration set value of the marker for the current time period, Gᵣₜ represents the real-time measured concentration of the marker for the current time period, V_{cs} represents a volume of a cell culture solution in a bioreactor, and Gₛᵤₚₚₗ represents a concentration of the marker in the supplemental culture medium; or
in a case that the rate of change of the marker is expressed as a generation rate,
   ${m}_{suppl} = k \times \left({A}_{rt}-{A}_{set}\right) \times {V}_{cs} / {B}_{suppl} ,$
where mₛᵤₚₚₗ represents the addition amount of the supplemental culture medium for the current time period, Aₛₑₜ represents the component concentration set value of the marker for the current time period, Aᵣₜ represents the real-time measured concentration of the marker for the current time period, V_{cs} represents the volume of the cell culture solution in the bioreactor, Bₛᵤₚₚₗ represents a concentration of a culture medium component, from which the marker is converted, in the supplemental culture medium, and k represents a conversion coefficient from the concentration of the marker to the concentration of the culture medium component.

In some embodiments, calculating the addition amount of the supplemental culture medium for the current time period based on the current rate of change of the marker includes one or more of:
in a case that the rate of change of the marker is expressed as a consumption rate,
   ${m}_{suppl} = R \times {V}_{cs} / {G}_{suppl} , R = {G}_{t 0} - {G}_{rt} ,$
where mₛᵤₚₚₗ represents the addition amount of the supplemental culture medium for the current time period, R is the current rate of change of the marker, V_{cs} represents a volume of a cell culture solution in a bioreactor, Gₛᵤₚₚₗ represents a concentration of the marker in the supplemental culture medium, Gₜ₀ represents a real-time measured concentration of the marker for a previous time period, and Gᵣₜ represents the real-time measured concentration of the marker for the current time period; or
in a case that the rate of change of the marker is expressed as a generation rate,
   ${m}_{suppl} = k \times R \times {V}_{cs} / {B}_{suppl} , R = {A}_{rt} - {A}_{t 0} ,$
where mₛᵤₚₚₗ represents the addition amount of the supplemental culture medium for the current time period, R is the current rate of change of the marker, V_{cs} represents the volume of the cell culture solution in the bioreactor, Aₜ₀ represents the real-time measured concentration of the marker for the previous time period, Aᵣₜ represents the real-time measured concentration of the marker for the current time period, Bₛᵤₚₚₗ represents a concentration of a culture medium component, from which the marker is converted, in the supplemental culture medium, and k represents a conversion coefficient from the concentration of the marker to the concentration of the culture medium component.

In some embodiments, the automatic material supplementing method includes: determining a material supplementing strategy to be applied based on the first material supplementing strategy and the second material supplementing strategy.

In some embodiments, the material supplementing strategy to be applied is determined to be an average of the first material supplementing strategy and the second material supplementing strategy.

In some embodiments, the material supplementing strategy to be applied is selected from the first material supplementing strategy and the second material supplementing strategy based on a phase into which the current time period falls.

In some embodiments, in a case that the current time period falls into a target product rapid production phase, the first material supplementing strategy is selected as the material supplementing strategy to be applied; and in a case that the current time period falls into the logarithmic growth phase, the second material supplementing strategy is selected as the material supplementing strategy to be applied.

In some embodiments, calculating the addition amount of the supplemental culture medium for the current time period includes:
calculating an addition amount of the marker or of a culture medium component, from which the marker is converted, for the current time period, based on the difference between the real-time measured concentration of the marker for the current time period and the component concentration set value of the marker for the current time period, or based on the current rate of change of the mark; and
calculating the addition amount of the supplemental culture medium for the current time period based on the addition amount of the marker or of the culture medium component, from which the marker is converted, for the current time period.

In some embodiments, the supplemental culture medium comprises a specific proportion of the marker and another component, and an addition amount of the other component is obtained based on an addition amount of the marker.

In some embodiments, the automatic material supplementing method includes writing the material supplementing strategy into a control system, where the control system is configured to perform material supplementing instantaneously in a dose-wise manner by cooperating with a peristaltic pump hardware upon receiving an numerical instruction on the material supplementing strategy.

In some embodiments, a real-time measured concentration of a reference component for the current time period is compared with a set value, and the automatic material supplementing method is not performed responsive to the real-time measured concentration of the reference component for the current time period being not lower than the set value, or the automatic material supplementing method is performed responsive to the real-time measured concentration of the reference component for the current time period being lower than the set value .

In some embodiments, the current real-time dynamic measurement result is online spectral data, and calculating the real-time measured concentration of the marker for the current time period based on the current real-time dynamic measurement result includes inputting the current real-time dynamic measurement result into a trained text convolutional model to obtain the real-time measured concentration of the marker for the current time period, where the text convolution model is trained by taking the online spectral data of an indicator of a cell culture solution in a bioreactor at multiple different time points as samples and taking offline target values of the indicator measured by sampling the cell culture solution as labels, and the text convolution model is configured to receive real-time measured spectral data of the indicator of a cell culture solution to be predicted and output a prediction value of the indicator.

In some embodiments, the text convolution model is configured to: set a word vector mapping layer and perform feature dimensionality reduction; and obtain, using a fully connected structure, the prediction value of the indicator after averaging over a spatial dimension.

In some embodiments, the text convolution model is configured to: further perform neighbor feature sampling after setting the word vector mapping layer and performing the feature dimensionality reduction.

In some embodiments, the online spectral data is Raman spectral signal data.

In some embodiments, the indicator includes one or more of: pH value, carbon dioxide partial pressure, sodium ion concentration, potassium ion concentration, glucose concentration, lactate concentration, ammonium ion concentration, glutamine concentration, glutamate concentration, target protein concentration, lactate dehydrogenase concentration, terbium ion concentration, phosphate ion concentration, osmotic pressure, viable cell density, cell viability, average diameter of viable cells, and amino acid concentration.

In some embodiments, calculating the real-time measured concentration of the marker for the current time period based on the current real-time dynamic measurement result comprises inputting the current real-time dynamic measurement result into a machine learning ensemble model for predicting a component concentration in a cell culture solution to obtain the real-time measured concentration of the marker for the current time period,
where the machine learning ensemble model is built by:
obtaining a dataset of a component concentration of a cell culture solution in a bioreactor, the dataset including a training dataset, a validation dataset, and a test dataset;
building multiple single prediction models by using multiple machine learning algorithms, respectively, where each of the multiple single prediction models performs prediction on the test dataset after being trained with the training dataset and validated with the validation dataset;
obtaining a square sum of prediction errors of the multiple single prediction models by comparing prediction results of the multiple single prediction models with the test dataset, and determining weights of the multiple single prediction models in the machine learning ensemble model based on a magnitude of the square sum of the prediction errors; and
obtaining the machine learning ensemble model by combining the multiple single prediction models through a weight assignment method.

In some embodiments, calculating the real-time measured concentration of the marker for the current time period based on the current real-time dynamic measurement result comprises inputting the current real-time dynamic measurement result into a machine learning ensemble model for predicting a component concentration in a cell culture solution to obtain the real-time measured concentration of the marker for the current time period,
where the machine learning ensemble model is a transferred model obtained by performing model transfer relative to an original model,
where the original model is built by:
obtaining a dataset of a component concentration of a cell culture solution in a bioreactor, the dataset comprising a training dataset, a validation dataset, and a test dataset;
building multiple single prediction models by using multiple machine learning algorithms, respectively, where each of the multiple single prediction models performs prediction on the test dataset after being trained with the training dataset and validated with the validation dataset;
obtaining a square sum of prediction errors of the multiple single prediction models by comparing prediction results of the multiple single prediction models with the test dataset, and determining weights of the multiple single prediction models in the original model based on a magnitude of the square sum of the prediction errors; and
obtaining the original model by combining the multiple single prediction models through a weight assignment method;
where the model transfer includes:
   obtaining an original dataset used for building the original model, and a new-batch training dataset of the component concentration of a cell culture solution of a new batch of biological reactions, the original dataset including an original training dataset, an original validation dataset, and an original test dataset,
   obtaining a new training dataset by performing scale correction or scale matching on the new-batch training dataset and the original training dataset,
   building multiple single prediction models by using multiple machine learning algorithms, respectively, with the new training dataset, the original validation dataset, and the original test dataset, where each of the multiple single prediction models performs prediction on the original test dataset after being trained with the new training dataset and validated with the original validation dataset,
   obtaining a square sum of prediction errors of the multiple single prediction models by comparing prediction results of the multiple single prediction models with the original test dataset, and determining weights of the multiple single prediction models in the transferred model based on a magnitude of the square sum of the prediction errors; and
   obtaining the transferred model by combining the multiple single prediction models through a weight assignment method,
   where the scale correction includes incorporating a specified proportion of data from the new-batch training dataset into the original training dataset used for building the original model; and
   the scale matching includes incorporating new-batch training data, with a numerical difference from original training data collected at an identical time point as the new-batch training data in the original training dataset being less than a specified threshold, from the new-batch training dataset into the original training dataset.

In some embodiments, the specified proportion is a value selected from 1% to 10%, or a value selected from 1.5% to 7.5%, or a value selected from 2% to 5%. In some embodiments, the specified threshold is a value selected from 1% to 10%, or a value selected from 3% to 8%, or a value selected from 4% to 6%, or 5%.

In some embodiments, the multiple machine learning algorithms include at least two selected from: Partial Least Squares, Cubist, Random Forest, Support Vector Machine, or Time Series.

In some embodiments, the dataset of the component concentration comprises online Raman spectral data and corresponding offline measurement data thereof, and a sampling time point of the offline measurement data is matched with corresponding online Raman spectral data.

In some embodiments, the component concentration is selected from: viable cell density, glucose concentration, lactate concentration, target product concentration, and amino acid concentration.

In some embodiments, the method further includes data preprocessing of the Raman spectral data, the data preprocessing including at least one of: screening out abnormal data points, removing spikes, Raman shift correction and light intensity correction, baseline calibration, smoothing, or differentiation.

In some embodiments, selecting one component or the combination of multiple components as the marker based on the historical rate of change of each component includes:
taking one component or a combination of multiple components among N components with the highest historical rate of changes as the marker, where N is a positive integer.

In a fourth aspect, the present disclosure provides a non-transitory computer-readable storage medium storing a computer program thereon, where the computer program, when executed by a processor, implements steps of the automatic material supplementing method based on the real-time dynamic measurement result according to the first aspect or the third aspect.

In a fifth aspect, the present disclosure provides a computer device including a memory and a processor, where the memory stores thereon a computer program that is able to run on the processor, and the computer program, when executed by the processor, implements steps of the automatic material supplementing method based on the real-time dynamic measurement result according to the first aspect or the third aspect.

In a sixth aspect, the present disclosure provides a computer program product including instructions which, when executed by a processor, implement the automatic material supplementing method based on the real-time dynamic measurement result according to the first aspect or the third aspect.

### BRIEF DESCRIPTION OF THE DRAWINGS

The foregoing and other features and advantages of the present disclosure will become clear from the following description of embodiments of the present disclosure illustrated in conjunction with the accompanying drawings. The accompanying drawings, incorporated herein and forming part of the specification, are used to further explain the principles of the present disclosure and to enable those skilled in the art to make and use the present disclosure.
FIG. 1 is a flowchart of an automatic material supplementing method.
FIG. 2 is a flowchart of an feedback control application.
FIG. 3 is a diagram showing manual timed material supplementing.
FIG. 4 is a diagram showing real-time dynamic material supplementing.
FIG. 5 is a diagram showing a contrast effect of viable cell density.
FIG. 6 is a diagram showing a contrast effect of cell viability.
FIG. 7 is a diagram showing a contrast effect of glucose concentration.
FIG. 8 is a diagram showing a contrast effect of lactate concentration.
FIG. 9 is a diagram showing a contrast effect of lactate dehydrogenase concentration.
FIG. 10 is a diagram showing a contrast effect of target protein expression levels.
FIG. 11 is a diagram showing a contrast effect of protein expression levels in a single cell.
FIG. 12 is a diagram showing a contrast effect of ammonium ion concentration.
FIG. 13 is a diagram showing a contrast effect (I) of amino acid concentrations between manual and dynamic material supplementing when a marker is glucose.
FIG. 14 is a diagram showing a contrast effect (II) of amino acid concentrations between manual and dynamic material supplementing when a marker is glucose.
FIG. 15 is a diagram showing a contrast effect of viable cell density.
FIG. 16 is a diagram showing a contrast effect of cell viability.
FIG. 17 is a diagram showing a contrast effect of glucose concentration.
FIG. 18 is a diagram showing a contrast effect of lactate concentration.
FIG. 19 is a diagram showing a contrast effect of target protein concentration.
FIG. 20 is a schematic diagram of a control apparatus for cell culture material supplementing.
FIG. 21 is a flowchart showing an automatic material supplementing method based on a real-time dynamic measurement result according to some embodiments of the present disclosure.
FIG. 22 is a flowchart showing an automatic material supplementing method based on a real-time dynamic measurement result according to some embodiments of the present disclosure.
FIG. 23 is a schematic block diagram showing an automatic material supplementing apparatus based on a real-time dynamic measurement result according to some embodiments of the present disclosure.
FIG. 24 is a schematic block diagram showing an automatic material supplementing apparatus based on a real-time dynamic measurement result according to some embodiments of the present disclosure.
FIG. 25 is a schematic diagram of training and application of a text convolutional model according to some embodiments of the present disclosure.
FIG. 26 is a schematic diagram of training and application of a machine learning ensemble model according to some embodiments of the present disclosure.
FIG. 27 is a flowchart of data preprocessing according to some embodiments of the present disclosure.
FIG. 28 is a workflow diagram of data learning according to some embodiments of the present disclosure.
FIG. 29 shows two methods for model transfer according to some embodiments of the present disclosure.
FIG. 30 shows a prediction effect of viable cell density.
FIG. 31 shows a prediction effect of glucose concentration.
FIG. 32 shows a prediction effect of lactate concentration.
FIG. 33 shows a prediction effect of osmotic pressure.
FIG. 34 shows a prediction effect of target protein concentration.
FIG. 35 shows a prediction effect of histidine concentration.
FIG. 36 shows a prediction effect of viable cell density of a model when transferring across different scales.
FIG. 37 shows a prediction effect of glucose concentration of a model when transferring across different scales.
FIG. 38 shows a schematic block diagram of a computer device according to some embodiments of the present disclosure;
FIG. 39 shows a schematic block diagram of a computer system on which embodiments of the present disclosure may be implemented.

Note that in the embodiments described below, the same reference numerals are sometimes used in different drawings to indicate the same part or part with the same function, and repeated descriptions are omitted. In some cases, similar labels and letters are used to denote similar items, so once an item is defined for one drawing, it does not need to be further discussed for subsequent drawings.

For ease of understanding, the positions, dimensions, and ranges of the structures shown in the accompanying drawings and the like may not represent the actual positions, dimensions, and ranges, etc. Therefore, the present disclosure is not limited to the positions, dimensions, and ranges disclosed in the accompanying drawings and the like.

### DETAILED DESCRIPTION

Various exemplary embodiments of the present disclosure will be described below in detail with reference to the accompanying drawings. It should be noted that, unless otherwise specifically stated, the relative arrangement, numerical expressions, and values of the components and steps set forth in these embodiments do not limit the scope of the present disclosure.

The following description of at least one exemplary embodiment is merely illustrative and is in no way intended to limit the present disclosure or its application or use. In other words, the structures and methods described herein are illustrated in an exemplary manner to illustrate different embodiments of the structures and methods in the present disclosure. However, those skilled in the art will understand that these embodiments are merely illustrative of exemplary ways in which the present disclosure may be implemented, and not exhaustive. Furthermore, the accompanying drawings need not be drawn to scale, and some features may be enlarged to show details of specific components.

In addition, technologies, methods and devices known to an ordinary skilled person in the related art may not be discussed in detail, but where appropriate, such technologies, methods and devices should be considered part of the specification.

In all the examples shown and discussed herein, any specific values should be interpreted as merely exemplary, not as limitations. Therefore, other examples of the exemplary embodiments may have different values.

An automatic material supplementing method based on a real-time dynamic measurement result according to various embodiments of the present disclosure is described in detail below with reference to the accompanying drawings. It should be understood that the actual method may include other additional steps, but in order to avoid obscuring the key points of the present disclosure, these additional steps will not be discussed herein and will not be shown in the accompanying drawings.

Currently, in order to maintain nutrients required for cell growth environments during cell culture, it is necessary to perform material supplementing of a cell culture solution. However, an addition amount of a supplemental culture medium and a time point for material supplementing is often determined based on experiences on related processes, which may lead to insufficient or excessive supplemental amount within a unit time, as well as inconsistent time points for material supplementing, affecting the development of processes and ultimately resulting in yield variations between batches.

The cell culture solution is a critical factor affecting the success of cell culture, and has a significant impact on how to regulate cell culture process parameters. Biopharmaceutical research and development requires a large number of cell culture experiments, and the most reliable cell culture process is selected through experimental results to ensure a stable production flow and yield in the long term. Ideally, cell culture experiments should be able to explore the effects of concentrations of a wide range of cell culture solution components (such as sugars, metal ions, or amino acids) on cell lines at every moment. However, this is a challenge for the entire biopharmaceutical industry, requiring not only a significant investment of time, manpower, and material resources, but also very high technical capabilities. The application of Raman spectroscopy analysis models enables real-time and rapid monitoring of various parameters during cell culture, which can shorten process development time and save experimental costs in small-scale laboratory process development stages; and reduce batch-to-batch differences in product quality and yield in large-scale production stages.

The operation of material supplementing during the cell culture process is a critical step of the entire process, and has an irreversible and significant impact on the cell culture process. Therefore, developing a real-time dynamic automatic material supplementing strategy has significant application value.

To address one or more of the above-described problems, the present disclosure provides an automatic material supplementing method based on a real-time dynamic measurement result. In the present disclosure, the process analysis technology is adopted to provide real-time feedback of component concentrations of the cell culture solution, enabling timely and on-demand dynamic material supplementing in a manner of automatic feedback without human intervention. This solves the problem of complex and inefficient process development strategies in the production of recombinant proteins through cell culture, which relies on a large number of traditional material supplementing parallel experiments and manual phased sampling methods.

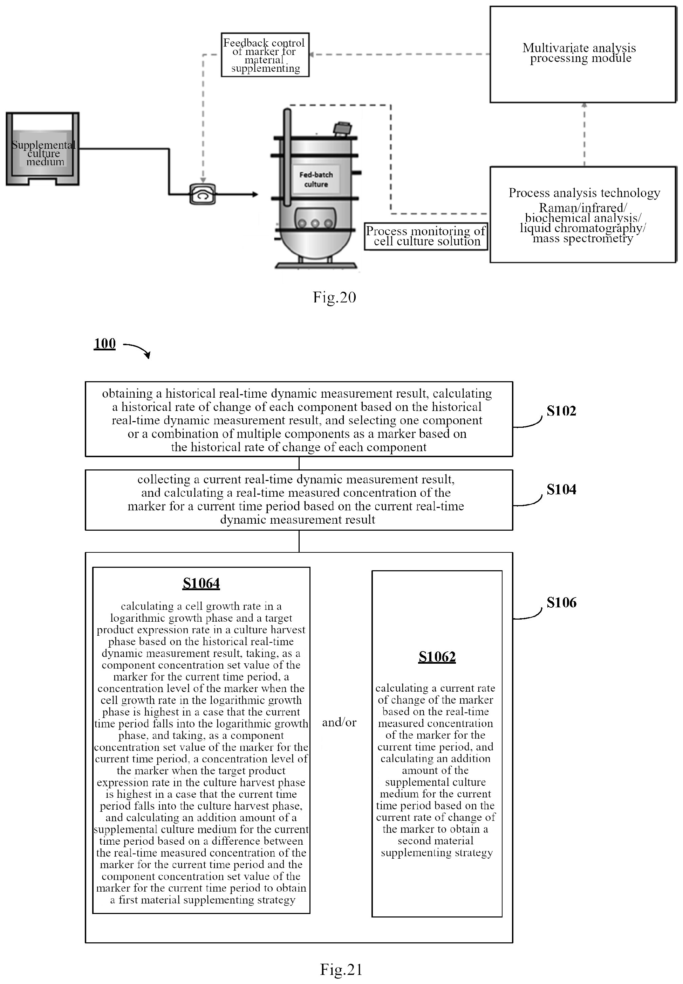

Referring to FIG. 21, an automatic material supplementing method 100 based on a real-time dynamic measurement result (hereinafter briefly referred to as the method 100) according to some embodiments of the present disclosure is shown. The method 100 includes steps S102 to S106.

In step S102, a historical real-time dynamic measurement result is obtained, a historical rate of change of each component is calculated based on the historical real-time dynamic measurement result, and one component or a combination of multiple components is selected as a marker based on the historical rate of change of each component.

Herein, the rate of change may indicate a change in a concentration within a unit time. It can be understood that, depending on a specific type of a component, the rate of change of the component may be expressed as a consumption rate or a generation rate. For example, for consumable components such as nutrients like glucose, the rate of change thereof is usually expressed as the consumption rate, while for generative components such as products like target protein, the rate of change thereof is usually expressed as the generation rate. For the sake of simplicity, the consumption rate and the generation rate are collectively referred to as the rate of change herein, and those skilled in the art can clearly identify whether the rate of change is the consumption rate or the generation rate based on whether a specific component is a consumable component or a generative component.

In some embodiments, calculating the historical rate of change of each component based on the historical real-time dynamic measurement result may include: calculating a real-time measured concentration of each component for a historical time period based on the historical real-time dynamic measurement result, and calculating the historical rate of change of each component based on the real-time measured concentration of each component for the historical time period. Various exemplary implementations of calculating the real-time measured concentration of each component (including the marker) based on the real-time dynamic measurement result will be described later in details.

In some embodiments, step S102 may include: calculating multiple historical rates of change of each component within multiple historical time periods based on the historical real-time dynamic measurement result; and combining the multiple historical rates of change of each component to obtain a combined historical rate of change of each component, and selecting one component or a combination of multiple components as the marker based on the combined historical rate of change of each component.

For example, during an experiment from which real-time dynamic measurement results are collected, each 5-minute interval may be taken as one time period, so that there may be multiple such 5-minute time periods during an experiment, for example, lasting 14 days. The rate of change of a component in each time period may be determined by comparing a real-time measured concentration of the component for the time period with a real-time measured concentration of the component for a previous time period. For example, rates of change of the component within multiple time periods may be combined in any suitable way (including but not limited to averaging, weighted averaging, finding the maximum value, finding the minimum value, finding the median, etc.) to obtain a combined rate of change of the component. Then, the marker may be selected based on the combined rate of change.

In some embodiments, selecting one component or a combination of multiple components as the marker based on the historical rate of change of each component may include: taking one component or a combination of multiple components among N components having the highest historical rate of change as the marker, where N is a positive integer. In some examples, N = 1. In some examples, N>1, for example, N = 2, 3, 5, 7, or 10. In some implementations, the components may be ranked from fastest to slowest according to their historical rates of change, and one component or a combination of multiple components among some top-ranked components is selected as the marker. The component selected as the marker may or may not include a component having the highest historical rate of change. In some implementations, each component and each combination of components in these top-ranked components may be taken as candidate markers respectively for culture control experiments, and the candidate marker that performs better in the culture control experiments is ultimately determined as the marker, so that the maker may be used to determine a material supplementing strategy in a subsequent culture process.

In step S104, the current real-time dynamic measurement results are collected, and the real-time measured concentration of the marker for the current time period is calculated based on the current real-time dynamic measurement results. As mentioned above, various exemplary implementations for calculating the real-time measured concentration of components (such as markers) based on real-time dynamic measurement results will be described in detail below.

In step S106, a material supplementing strategy is obtained through at least one of sub-steps S1062 or S1064.

Specifically, in sub-step S1062, a cell growth rate in a logarithmic growth phase and a target product expression rate in a culture harvest phase are calculated based on the historical real-time dynamic measurement result, a concentration level of the marker when the cell growth rate in the logarithmic growth phase is highest is taken as a component concentration set value of the marker for the current time period in a case that the current time period falls into the logarithmic growth phase, a concentration level of the marker when the target product expression rate in the culture harvest phase is highest is taken as a component concentration set value of the marker for the current time period in a case that the current time period falls into the culture harvest phase, and an addition amount of a supplemental culture medium for the current time period is calculated based on a difference between the real-time measured concentration of the marker for the current time period and the component concentration set value of the marker for the current time period to obtain a first material supplementing strategy. For example, the historical real-time dynamic measurement result and the current real-time dynamic measurement result may be obtained from different fermentation processes with the same cell culture process mode. Specifically, the historical real-time dynamic measurement result may be collected in a historical fermentation process, and the current real-time dynamic measurement result may be collected during a present fermentation process, while the historical and present fermentation processes may have the same cell culture process mode.

As a non-limiting example, calculating the addition amount of the supplemental culture medium for the current time period based on the difference between the real-time measured concentration of the marker for the current time period and the component concentration set value of the marker for the current time period may include one or more of:
in a case that the rate of change of the marker is expressed as a consumption rate, in other words, in a case that the marker is a consumable component,
   ${m}_{suppl} = \left({G}_{set}-{G}_{rt}\right) \times {V}_{cs} / {G}_{suppl} ,$
where mₛᵤₚₚₗ represents the addition amount of the supplemental culture medium for the current time period, Gₛₑₜ represents the component concentration set value of the marker for the current time period, Gᵣₜ represents the real-time measured concentration of the marker for the current time period, V_{cs} represents a volume of a cell culture solution in a bioreactor, and Gₛᵤₚₚₗ represents a concentration of the marker in the supplemental culture medium; or
in a case that the rate of change of the marker is expressed as a generation rate, in other words, in a case that the marker is a generative component,
   ${m}_{suppl} = k \times \left({A}_{rt}-{A}_{set}\right) \times {V}_{cs} / {B}_{suppl} ,$
where mₛᵤₚₚₗ represents the addition amount of the supplemental culture medium for the current time period, Aₛₑₜ represents the component concentration set value of the marker for the current time period, Aᵣₜ represents the real-time measured concentration of the marker for the current time period, V_{cs} represents the volume of the cell culture solution in the bioreactor, Bₛᵤₚₚₗ represents a concentration of a culture medium component, from which the marker is converted, in the supplemental culture medium, and k represents a conversion coefficient from the concentration of the marker to the concentration of the culture medium component.

It is understood that the generative component is generally converted from one or more culture medium components. Therefore, the addition amount of the supplemental culture medium may be calculated based on the coefficient of the concentration relationship between the generative component and the one or more culture medium components.

In sub-step S1064, an addition amount of the supplemental culture medium for the current time period is calculated based on the current rate of change of the marker to obtain a second material supplementing strategy.

As a non-limiting example, calculating the addition amount of the supplemental culture medium for the current time period based on the current rate of change of the marker may include one or more of:
in a case that the rate of change of the marker is expressed as a consumption rate, in other words, in a case that the marker is a consumable component,
   ${m}_{suppl} = R \times {V}_{cs} / {G}_{suppl} , R = {G}_{t 0} - {G}_{rt} ,$
where mₛᵤₚₚₗ represents the addition amount of the supplemental culture medium for the current time period, R is the current rate of change of the marker, V_{cs} represents a volume of a cell culture solution in a bioreactor, Gₛᵤₚₚₗ represents a concentration of the marker in the supplemental culture medium, Gₜ₀ represents a real-time measured concentration of the marker for a previous time period, and Gᵣₜ represents the real-time measured concentration of the marker for the current time period; or
in a case that the rate of change of the marker is expressed as a generation rate, in other words, in a case that the marker is a generative component,
   ${m}_{suppl} = k \times R \times {V}_{cs} / {B}_{suppl} , R = {A}_{rt} - {A}_{t 0} ,$
where mₛᵤₚₚₗ represents the addition amount of the supplemental culture medium for the current time period, R is the current rate of change of the marker, V_{cs} represents the volume of the cell culture solution in the bioreactor, Aₜ₀ represents the real-time measured concentration of the marker for the previous time period, Aᵣₜ represents the real-time measured concentration of the marker for the current time period, Bₛᵤₚₚₗ represents a concentration of a culture medium component, from which the marker is converted, in the supplemental culture medium, and k represents a conversion coefficient from the concentration of the marker to the concentration of the culture medium component.

Sub-steps S1062 and S1064 may be used individually or in combination. When used in combination, one of sub-steps S1062 and S1064 may be selected according to conditions, such as in stages; or an average value, weighted value or otherwise-combined value of the supplemental amounts calculated by using the sub-steps S1062 and S1064 respectively may be used.

Therefore, in some embodiments, the method 100 may include determining a material supplementing strategy to be applied based on the first material supplementing strategy and the second material supplementing strategy. In some examples, the material supplementing strategy to be applied is determined to be an average of the first material supplementing strategy and the second material supplementing strategy. In some other examples, the material supplementing strategy to be applied is selected from the first material supplementing strategy and the second material supplementing strategy based on a phase into which the current time period falls. For example, in a case that the current time period falls into a target product rapid production phase (e.g., protein rapid production phase), the first material supplementing strategy is selected as the material supplementing strategy to be applied; and in a case that the current time period falls into the logarithmic growth phase, the second material supplementing strategy is selected as the material supplementing strategy to be applied.

Specifically, calculating the addition amount of the supplemental culture medium for the current time period may include: calculating an addition amount of the marker or of a culture medium component, from which the marker is converted, for the current time period, based on the difference between the real-time measured concentration of the marker for the current time period and the component concentration set value of the marker for the current time period, or based on the current rate of change of the mark; and calculating the addition amount of the supplemental culture medium for the current time period based on the addition amount of the marker or of the culture medium component, from which the marker is converted, for the current time period. In other words, no matter whether in sub-step S 1062 or S 1064, the addition amount of the marker or of the culture medium component, from which the marker is converted, for the current time period may be calculated first, and then the addition amount of the supplemental culture medium for the current time period may be calculated based on the addition amount of the marker or of the culture medium component, from which the marker is converted, for the current time period.

For the marker that is a consumable component, the addition amount of the supplemental culture medium for the current time period may be calculated directly based on the addition amount of the marker for the current time period. For example, the supplemental culture medium may include a specific proportion of the marker and another component, thereby allowing the addition amount of the other component to be determined based on the addition amount of the marker. For the marker that is a generative component, the addition amount of the culture medium component from which the marker is converted may be determined first based on the change of the concentration of the marker, and then the addition amount of the supplemental culture medium for the current time period may be calculated based on the addition amount of the culture medium component from which the marker is converted.

In some embodiments, the method 100 may further include writing the material supplementing strategy into a control system. The control system may be configured to perform material supplementing instantaneously in a dose-wise manner by cooperating with a peristaltic pump hardware upon receiving an numerical instruction on the material supplementing strategy.

For example, the real-time dynamic measurement result may be collected every time period (e.g., 5 minutes) and the real-time measured concentration of each component for this time period may be calculated based on the real-time dynamic measurement result collected for this time period. One or more of the components or all of the components may be selected as reference components. For example, the marker selected as described above or the culture medium component from which the marker is converted may be taken as the reference component. The real-time measured concentration of the reference component for the current time period is compared with a set value, and whether to perform the method 100 is determined based on the result of the comparison. For example, responsive to that the real-time measured concentration of the reference component for the current time period is not lower than the set value (indicating that the material supplementing is not needed at this time), the method 100 is not performed (thereby saving computational resources); or responsive to that the real-time measured concentration of the reference component for the current time period is lower than the set value (indicating that the material supplementing may be needed at this time), the method 100 is performed (thereby timely supplementing materials). The set value here may be specifically set according to actual needs, which may be taken as different values for different components, and may be expressed not only as a single value, but also as multiple values or ranges.

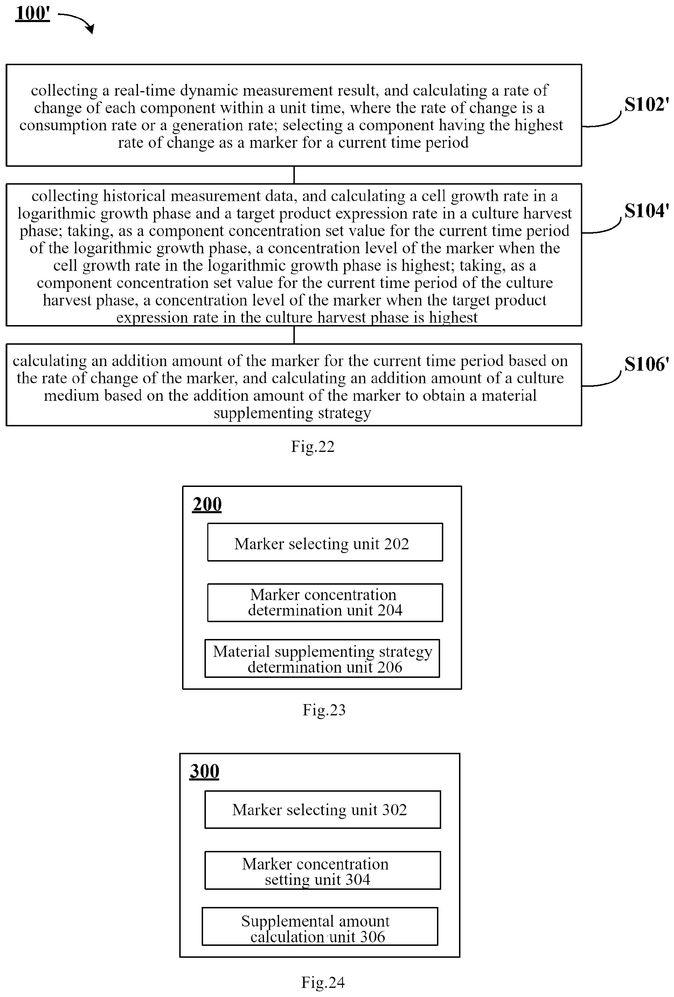

FIG. 22 shows an automatic material supplementing method 100' (hereinafter briefly referred to as the method 100') based on a real-time dynamic measurement result according to some embodiments of the present disclosure. The method 100' includes steps S102' to S106'.

In step S102', a real-time dynamic measurement result is collected, and a rate of change of each component within a unit time is calculated, where the rate of change is a consumption rate or a generation rate; a component having the highest rate of change is selected as a marker for a current time period.

In step S104', historical measurement data is collected, and a cell growth rate in a logarithmic growth phase and a target product expression rate in a culture harvest phase are calculated; a concentration level of the marker when the cell growth rate in the logarithmic growth phase is highest is taken as a component concentration set value for the current time period of the logarithmic growth phase; a concentration level of the marker when the target product expression rate in the culture harvest phase is highest is taken as a component concentration set value for the current time period of the culture harvest phase.

In step S106', an addition amount of the marker for the current time period is calculated based on the rate of change of the marker, and an addition amount of a culture medium is calculated based on the addition amount of the marker to obtain a material supplementing strategy.

In some embodiments, step S102' may include: collecting a real-time dynamic measurement result, and calculating a real-time measured concentration of each component for the current time period based on the real-time dynamic measurement result; calculating the rate of change of each component within the unit time based on the real-time measured concentration of each component for the current time period, and selecting one component or a combination of multiple components (the selection manner may be similar with reference to the above embodiments of the method 100) as the marker for the current time period based on the rate of change of each component. In some embodiments, step S104' may include: collecting historical measurement data, calculating a cell growth rate in a logarithmic growth phase and a target product expression rate in a culture harvest phase; taking, as a component concentration set value of the marker for the current time period, a concentration level of the marker when the cell growth rate in the logarithmic growth phase is highest in a case that the current time period falls into the logarithmic growth phase; and taking, as a component concentration set value of the marker for the current time period, a concentration level of the marker when the target product expression rate in the culture harvest phase is highest in a case that the current time period falls into the culture harvest phase. In some embodiments, step S106' may alternatively include calculating an addition amount of a supplemental culture medium for the current time period based on a difference between the real-time measured concentration of the marker for the current time period and the component concentration set value of the marker for the current time period, and/or based on the rate of change of the marker.

The difference between the method 100' and the method 100 is that, the method 100 statically selects the marker for material supplementing control of the current culture process based on the historical measurement data, while the method 100' dynamically selects the marker for material supplementing control of the current culture process based on the current measurement data. The method 100 is conducive to achieving stable and reliable material supplementing control, while the method 100' is conducive to achieving flexible and adaptive material supplementing control. Other various embodiments of the method 100' are similar to those of the method 100, and therefore the above description may be referred to, which will not be elaborated further here.

As described above, the cell cultivation is now widely used in the research and development and production of biopharmaceuticals. Maintaining an ideal level of nutrients during each phase of the culture process is crucial, as it greatly affects the yield, quality and input cost of biopharmaceuticals. The optimization of methods for regulating the material supplementing is very important to ensure that cells grow in the optimal environment. Traditional material supplementing methods involve setting different material supplementing ratios based on experiences for the cultivation processes, and material supplementing is performed daily or every other day. This method not only requires a lot of manpower for sampling and measuring, and calculating the required supplemental amount based on offline measured data, but also requires devices (balance, peristaltic pump). Moreover, this material supplementing method cannot provide for monitoring in a timely manner, which may lead to nutrient deficiency or excess at some time in the cell culture process.

In contrast, in the present disclosure, consumption rates or generation rates of components are calculated based on (historical or current) time increment data, and a marker is selected from the components based on the consumption rates or generation rates of the components; a current measured concentration or a current consumption or generation rate of the marker is calculated based on the current time increment data, and a supplemental amount is dynamically calculated in real time based on the current measured concentration or the current consumption or generation rate of the marker. The present disclosure avoids large-scale sampling, reduces manual operation, and minimizes human error by feedback control for the operation of material supplementing by using the concentration of the marker in the cell culture solution. Meanwhile, by real-time monitoring and automatic control, it is ensured that the nutrient levels in the cell culture solution are maintained at a reasonable level actually needed by the cells throughout the entire culture process.

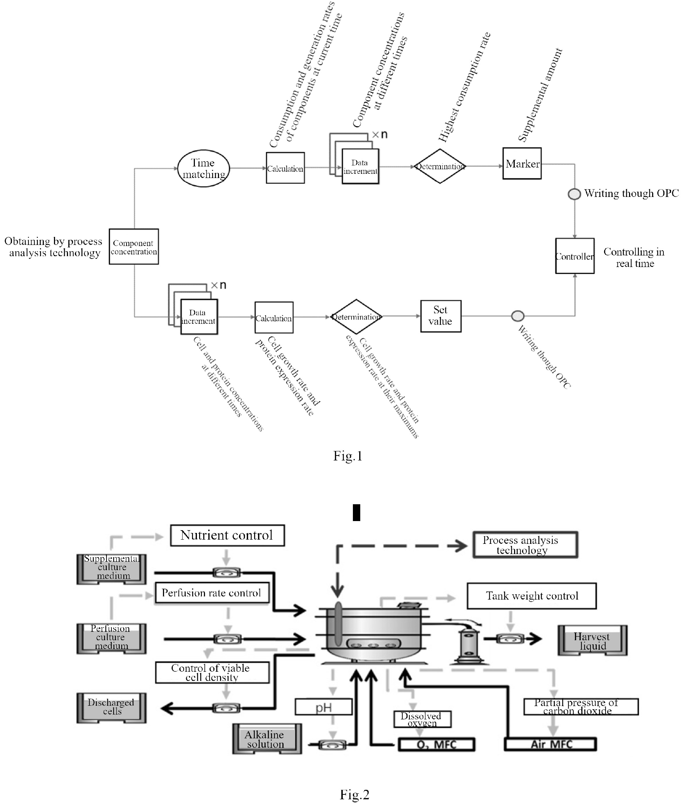

FIG. 1 is a flowchart showing a non-limiting example process in which an automatic material supplementing method according to an embodiment of the present disclosure is applied. As shown in FIG. 1, output parameters of a process analysis technology are first digitized and input into the software, matched with culture time, and a consumption/generation rate of each component within a unit time is calculated. The consumption/generation rates of different components within different time periods are calculated based on time increment data. One component or a combination of multiple components with the highest consumption/generation rate is selected as a marker. Based on the cell culture process mode and data, a cell growth rate in a logarithmic growth phase and a target protein expression rate in a culture harvest phase are calculated, respectively. When these two parameters are at their maximum, concentration levels of respective components are recorded. The concentration level of the marker, when each of these two parameters is at its maximum, is selected as a component concentration set value of the marker when the current time period falls into the respective phase. When a supplemental culture medium contains the marker, an addition amount of the marker for the current time period is calculated based on the consumption rate of the marker, and amounts of other nutrient components to be added into the bioreactor are calculated based on a ratio between concentrations of respective components in the supplemental culture medium. When the supplemental culture medium does not contain the marker, an addition amount of a culture medium component, from which the marker is converted, for the current time period is calculated based on the generation rate of the marker, while the amounts of other nutrient components to be added into the bioreactor are calculated based on the ratio between concentrations of respective components in the supplemental culture medium. Then, all numerical values of the material supplementing strategy are written into a control system through Object Linking and Embedding (OLE) Process Control (OPC) to complete the operation.

The present disclosure further provides a control apparatus for cell culture material supplementing, which enables real-time control of cell culture material supplementing. Respective units in the apparatus perform the following operational steps.
(1) A concentration of a component of a cell culture solution fed back by a process analysis tool is taken as a marker for feedback control.
   1.1 The process analysis tool includes but not limited to a Raman spectrometer, an infrared spectrometer, an online biochemical analyzer, an online high-performance liquid chromatograph, or an online mass spectrometer.
   1.2 The marker for feedback control is selected using the methods described above.
(2) Component concentration values are mapped to process value positions in a control module of a controller via a data transmission protocol (OPC/Modbus).
   2.1 The controller includes but not limited to Finesse, GE, or STR.
(3) Logic for processing component concentrations with multivariate analysis is loaded into the control module to calculate a supplemental amount. Process characteristics are identified to determine a time point for material supplementing.
   3.1 The control module for multivariate analysis and processing is developed based on the following software, including but not limited to SIMCA, R studio, or Matlab.
(4) A correlation formula between the supplemental amount and the time point for material supplementing is set.
(5) All operations are completed based on the supplemental amount and the time point for material supplementing.
(6) Alternatively, an amount of nutrients required by the cell at a moment is calculated based on the new component concentration value input at that moment, while the calculated value is mapped to the peristaltic pump of the controller.
(7) All operations are completed to complete a fermentation control process, and parameters for fermentation process control are output.

FIG. 23 shows an automatic material supplementing apparatus 200 (briefly referred to as the apparatus 200) based on a real-time dynamic measurement result according to some embodiments of the present disclosure. The apparatus 200 can be used to implement any embodiment of the method 100 described above. Specifically, the apparatus 200 includes a marker selecting unit 202, a marker concentration determination unit 204, and a material supplementing strategy determination unit 206. The marker selecting unit 202 may be configured to obtain a historical real-time dynamic measurement result, calculate a historical rate of change of each component based on the historical real-time dynamic measurement result, and select one component or a combination of multiple components as a marker based on the historical rate of change of each component. The marker concentration determination unit 204 may be configured to collect a current real-time dynamic measurement result, and calculate a real-time measured concentration of the marker for a current time period based on the current real-time dynamic measurement result. The material supplementing strategy determination unit 206 may be configured to obtain a material supplementing strategy by at least one of: calculating a cell growth rate in a logarithmic growth phase and a target product expression rate in a culture harvest phase based on the historical real-time dynamic measurement result, taking, as a component concentration set value of the marker for the current time period, a concentration level of the marker when the cell growth rate in the logarithmic growth phase is highest in a case that the current time period falls into the logarithmic growth phase, and taking, as a component concentration set value of the marker for the current time period, a concentration level of the marker when the target product expression rate in the culture harvest phase is highest in a case that the current time period falls into the culture harvest phase, and calculating an addition amount of a supplemental culture medium for the current time period based on a difference between the real-time measured concentration of the marker for the current time period and the component concentration set value of the marker for the current time period to obtain a first material supplementing strategy; or calculating a current rate of change of the marker based on the real-time measured concentration of the marker for the current time period, and calculating an addition amount of the supplemental culture medium for the current time period based on the current rate of change of the marker to obtain a second material supplementing strategy.

Various embodiments of the apparatus 200 may be similar with reference to various embodiments of the method 100, and will not be elaborated further here.

FIG. 24 shows an automatic material supplementing apparatus 300 (briefly referred to as the apparatus 300) based on a real-time dynamic measurement result according to some embodiments of the present disclosure. The apparatus 300 may be used to implement any embodiment of the method 100' described above. Specifically, the apparatus 300 includes a marker selecting unit 302, a marker concentration setting unit 304, and a supplemental amount calculation unit 306. The marker selecting unit 302 may be configured to: collect a real-time dynamic measurement result, and calculate a rate of change of each component within a unit time, where the rate of change is a consumption rate or a generation rate; and select a component having the highest rate of change as a marker for a current time period. The marker concentration setting unit 304 may be configured to: collect historical measurement data, and calculate a cell growth rate in a logarithmic growth phase and a target product expression rate in a culture harvest phase; take, as a component concentration set value for the current time period of the logarithmic growth phase, a concentration level of the marker when the cell growth rate in the logarithmic growth phase is highest; take, as a component concentration set value for the current time period of the culture harvest phase, a concentration level of the marker when the target product expression rate in the culture harvest phase is highest. The supplemental amount calculation unit 306 may be configured to: calculate an addition amount of the marker for the current time period based on the rate of change of the marker, and calculate an addition amount of a culture medium based on the addition amount of the marker to obtain a material supplementing strategy. Various embodiments of the apparatus 300 may be similar with reference to various embodiments of the method 100', and will not be elaborated further here.

Compared with related art, the present disclosure has the following beneficial effects.

The methods and apparatuses described in the present disclosure can improve the monitoring and control of the cell culture process and respond to the dynamic changes in the nutritional needs of cells; adjust the addition of mixed supplement culture media according to the concentrations of the components in the cell culture environment; provide the best culture environment for the cells, stabilize the process to the greatest extent and increase the yield; intelligently create material supplementing strategies to improve efficiency; and perform automatic material supplementing operations according to the created material supplementing strategies to reduce human error and the risk of contamination. The methods and apparatuses improve the speed and accuracy of cell culture process development; provide customized supplement culture media for different cell lines, clones, and processes; and can be rapidly applied to process development and production of different scales.

The technical solutions of the present disclosure will be further illustrated below through specific embodiments. Those skilled in the art will understand that the embodiments described are merely used to facilitate understanding of the present disclosure and should not be construed as specific limitations thereof.

Where technologies or conditions are not specified in the embodiments, the technologies or conditions described in the literature in this field, or the product manual, shall be followed. Reagents or instruments used, whose manufacturers are not specified, are all conventional products that may be purchased through legitimate channels.

### Comparative Example 1

**In** this comparative example, a traditional manual timed material supplementing strategy is adopted. Traditional material supplementing operations are classified according to a supplemental amount or physicochemical properties of supplemental materials, and are generally classified into following three categories: "large material supplementing operation" in which the supplemental material is weakly acidic and the supplemental amount is relatively large, "small material supplementing operation" in which the supplemental material is alkaline and the supplemental amount is relatively small, and separate material supplementing operation for the glucose stock solution. The amount and time point of the large and small material supplementing operations are determined by statistical analysis based on the rate of nutrient consumption by cells obtained from cell culture experiments.

The manual timed material supplementing strategy in this comparative example is to perform material supplementing every other day, that is, to manually add the supplemental culture medium into the reactor on the 2nd, 4th, 6th, 8th, 10th and 12th days during the culture. The formula for calculating the supplemental amount is as follows:
on the 2nd, 4th, and 6th days, m_{Large-suppl}=V_{cs} × 3%; m_{Small-suppl}=V_{cs} × 0.3% ;
on the 8th, and 10th days, m_{Large-suppl}=V_{cs} × 4%; m_{Small-suppl}=V_{cs} × 0.4%; and
on the 12th day, m_{Large-suppl}=V_{cs} × 1%; m_{Small-suppl}=V_{cs} × 0.1%.

The strategy for glucose supplementation is generally based on experiences or experimental data, so as to control the glucose concentration within an appropriate range. The amount of glucose to be supplemented daily is calculated by measuring the residual glucose concentration in the cell culture solution using an offline biochemical measurement device. The formula for calculating the amount of glucose to be supplemented manually is as follows:
${m}_{glu - suppl} = \left({G}_{glu - set}-{G}_{glu - resid}\right) \times {V}_{cs} / {G}_{suppl} ,$
where m_{glu-suppl} represents the amount of glucose stock solution to be added, G_{glu-set} represents the set glucose concentration, G_{glu-resid} represents the current residual glucose concentration measured by the offline device, V_{cs} represents the volume of the cell culture solution in the bioreactor, and Gₛᵤₚₚₗ represents the glucose concentration in the glucose stock solution.

### Embodiment 1

The automatic material supplementing method based on the real-time dynamic measurement result is used for automatic material supplementing control in Case 1. At the same time, the comparative experiment is conducted using the traditional manual timed material supplementing strategy in Comparative Example 1.

In this embodiment, a flowchart of applied feedback control is shown in FIG. 2.

The flowchart of the feedback control covers a variety of cell culture processes, such as a fed-batch culture process (material supplementing in batches) and a perfusion culture process (continuous material supplementing). In both the processes, the process analysis technology may be used to monitor concentrations of components in the cell culture solution. After analysis and calculation of the concentrations, the method of the present disclosure is used to control operation control points of respective processes in the flowchart, which include, but are not limited to: the operation point for material supplementing in batches, the operation point for continuous material supplementing, the operation point for cell discharge (for stabilizing the perfusion process, a certain amount of cell solution needs to be discharged from the reactor in real time or at regular intervals), the operation point for pH control (alkali supplementing), the operation for ventilation control, and the operation for harvesting rate control.

The experimental materials and related experimental methods in Case 1 are shown below.

In this embodiment, a CHO-K1 cell line that produces monoclonal antibodies is used in the part of the cell culture experiment. The culture conditions for this cell line in the shaker (Kuhner) for the seed phase are as follows: 36.5°C, a rotational speed of 110 rpm, a CO₂ concentration level of 6%, and a self-developed medium (MagniCHO) as a culture medium to be used; and the culture conditions for this cell line in the production culture phase are as follows: a 3L reactor with an initial culture volume of 1.5L, a culture temperature of 36.5°C, a pH set value of 6.90±0.25, a dissolved oxygen saturation of 40%, an initial inoculation density of 1.0×10⁶ cells/mL, and also a self-developed medium (MagniCHO Feed1) as a supplemental culture medium. The materials are shown in Table 1.

**Table 1**

| Name of materials | Item number | Production supplier |
|---|---|---|
| MagniCHO | CR80262 | Merck |
| MagniCHO Feed 1 | CR80270 | Merck |

High-dimensional data analysis and logical calculations of the processes are implemented using a open-source programming tool R (version 4.0.5).

The automatic material supplementing method based on the real-time dynamic measurement result includes the following steps.
(1) Historical real-time dynamic measurement results collected during historical fermentation processes with the same cell culture process mode as a current fermentation process are obtained, and multiple historical consumption/generation rates of each component within multiple historical time periods are calculated; and the multiple historical consumption/generation rates of each component are combined to obtain a combined historical consumption/generation rate of each component, and the component with the highest combined historical consumption/generation rate is selected as a marker.
(2) Current real-time dynamic measurement results are collected during the current fermentation process, and a real-time measured concentration of the marker for a current time period is calculated based on the current real-time dynamic measurement results.
(3) A cell growth rate in a logarithmic growth phase and a target product expression rate in a culture harvest phase are calculated based on the historical real-time dynamic measurement results, a concentration level of each component when these two parameters are at their maximum is obtained through statistics, and the concentration level corresponding to the selected marker is taken as a component concentration set value for the current time period.
(4) An addition amount of the marker or of a culture medium component, from which the marker is converted, for the current time period is calculated, and an addition amount of the supplemental culture medium is calculated based on the addition amount of the marker or of the culture medium component, from which the marker is converted, to obtain a material supplementing strategy. The material supplementing strategy is written into a control system. Upon receiving numerical instructions on the supplemental amount, the set value, and the time point for material supplementing, the control system cooperates with the peristaltic pump hardware to perform material supplementing instantaneously in a dose-wise manner. Once the current operation is completed, the control operation is stopped until the next instruction of material supplementing is issued.

In a case that the marker is a consumable component, the step of calculating the addition amount of the supplemental culture medium includes:
${m}_{suppl} = \left({G}_{set}-{G}_{rt}\right) \times {V}_{cs} / {G}_{suppl} ,$
where mₛᵤₚₚₗ represents the amount of the supplemental culture medium to be added, Gₛₑₜ represents a set concentration of the marker, Gᵣₜ represents a current concentration of the marker measured by analyzing a Raman spectrum in real time with a model, V_{cs} represents a volume of a cell culture solution in a bioreactor, and Gₛᵤₚₚₗ represents a concentration of the marker in the supplemental culture medium.

In a case that the marker is a generative component, the step of calculating the addition amount of the supplemental culture medium includes:
${m}_{suppl} = k \times \left({A}_{rt}-{A}_{set}\right) \times {V}_{cs} / {B}_{suppl} ,$
where mₛᵤₚₚₗ represents the addition amount of the supplemental culture medium for the current time period, Aₛₑₜ represents the component concentration set value of the marker for the current time period, Aᵣₜ represents the real-time measured concentration of the marker for the current time period, V_{cs} represents the volume of the cell culture solution in the bioreactor, Bₛᵤₚₚₗ represents a concentration of a culture medium component, from which the marker is converted, in the supplemental culture medium, and k represents a conversion coefficient from the concentration of the marker to the concentration of the culture medium component.

Each material supplementing strategy is summarized to obtain the automatic material supplementing strategy for the current fermentation process.

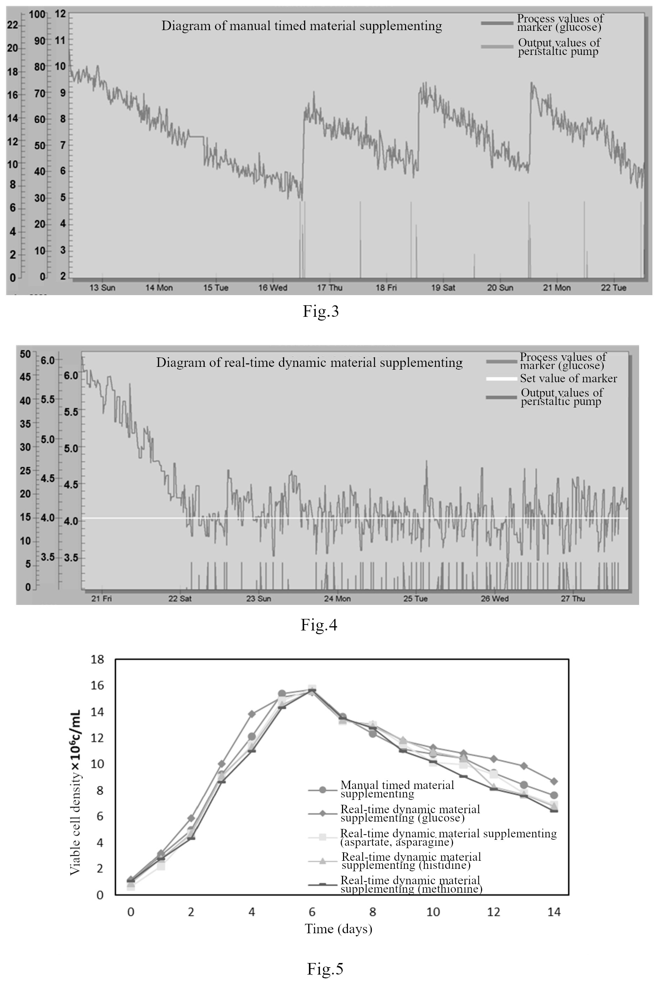

Based on the historical real-time dynamic measurement results collected during the historical fermentation processes, glucose (a consumable component) is selected as the marker for fermentation control. The amount and time point for supplementing glucose are shown in FIG. 3 and FIG. 4. FIG. 3 is a diagram showing the manual timed material supplementing, and FIG. 4 is a diagram showing the real-time dynamic material supplementing.

The schematic diagrams of operations of the manual timed material supplementing and the real-time dynamic material supplementing show that: a total of 7 operations of material supplementing occurred in the manual timed material supplementing process (indicated by the light-colored line segments at the bottom), and the corresponding marker (glucose) also changed in concentration at the moment of material supplementing (indicated by the dark-colored line segments at the top); and the real-time dynamic material supplementing distributes the supplemental amount evenly throughout the cell culture process, and the process values of the marker are always maintained near the set value.

The fermentation control is performed according to the automatic material supplementing strategy. In addition, the fermentation control is performed by taking other unselected components as markers for comparative experiments. The unselected components include aspartic acid, asparagine, histidine, methionine, or combinations thereof.

The fermentation results of the above automatic material supplementing strategy are shown below.

FIG. 5 is a diagram showing a contrast effect of viable cell density. The result indicates that the manual timed material supplementing operation and the automatic real-time dynamic material supplementing operations have no significant impact on the viable cell density in the cell culture process. The growth trends and peak density values of the viable cell density parameters under both material supplementing operations are comparable.

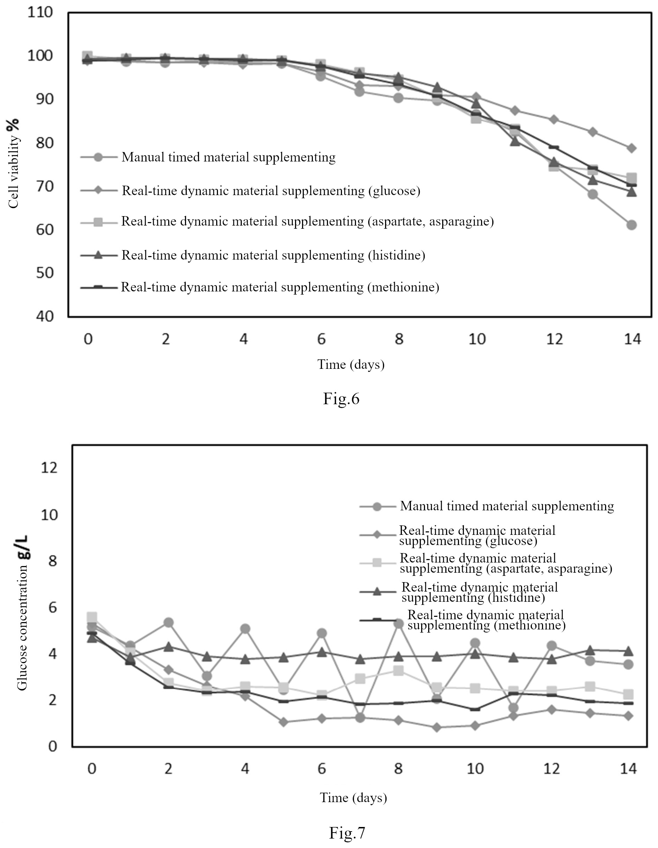

FIG. 6 is a diagram showing a contrast effect of cell viability. The result indicates that although the manual timed material supplementing can control glucose levels, the lactate concentration remains high due to long delays and fluctuating glucose concentration, resulting in low cell viability.

FIG. 7 is a diagram showing a contrast effect of glucose concentration. The result indicates that in the manual timed material supplementing, the supplemental amounts of material and glucose on the second or third day are regulated based on data from daily offline measurement. This method suffers from severe delays and significant fluctuations in glucose concentration in the cell culture solution.

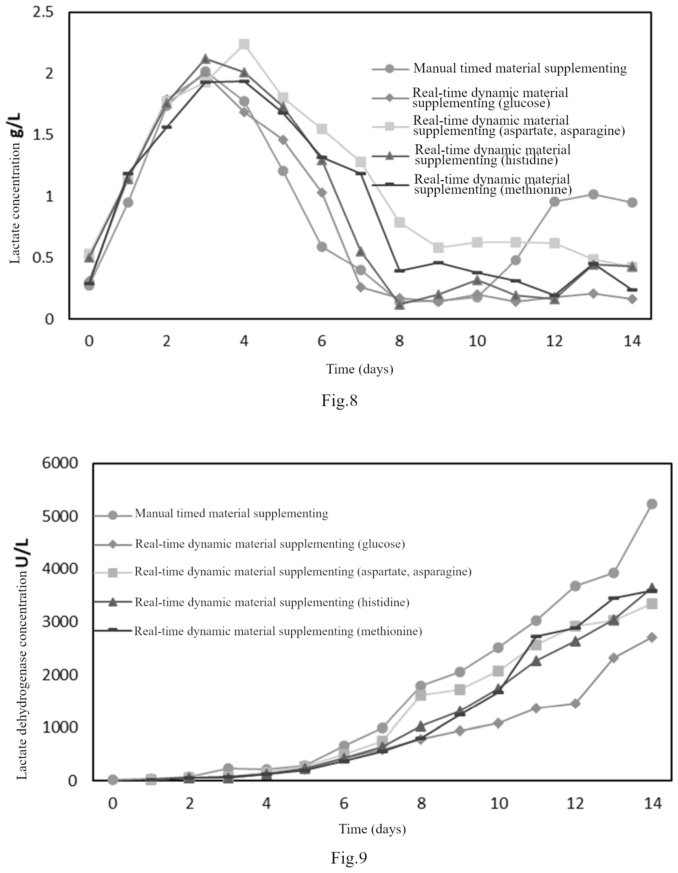

FIG. 8 is a diagram showing a contrast effect of lactate concentration. The result indicates that in the traditional method, the lactate is controlled by regulating the supplemental amounts of material and glucose on the second or third day based on data from daily offline measurement. The manual timed material supplementing has significant delays and the glucose concentration in the cell culture solution fluctuates greatly. Therefore, it is difficult to control lactate levels by controlling glucose concentration, resulting in lactate concentration remaining high.

FIG. 9 is a diagram showing a contrast effect of lactate dehydrogenase concentration. The result indicates that the lactate dehydrogenase concentration is higher in the traditional manual material supplementing operation than in the real-time dynamic material supplementing operations. The higher lactate concentration leads to a higher pH level in the cell culture solution, and the rapid decline in cell viability causes further deterioration of the culture medium condition, resulting in more cell death and rupture, and the release of more lactate dehydrogenase.

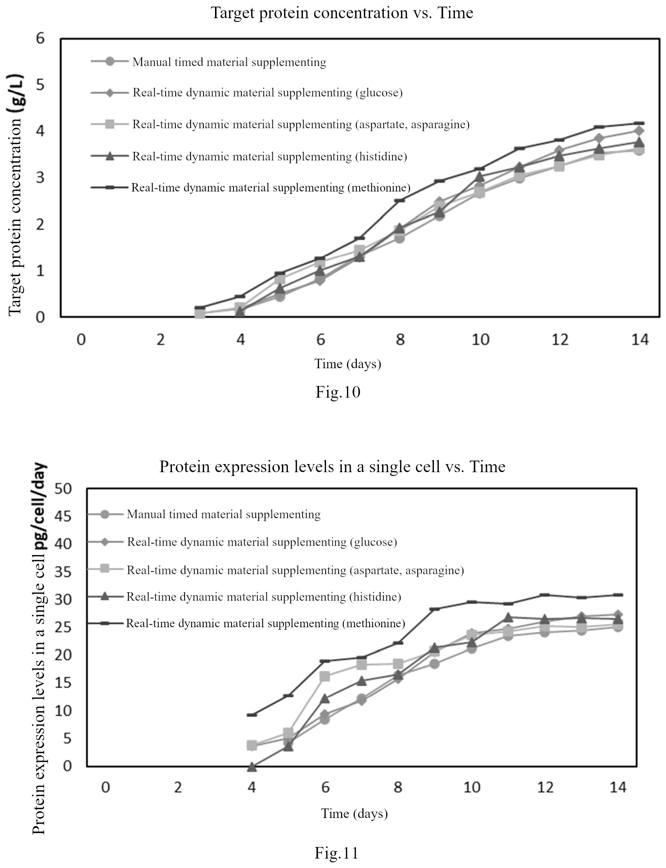

FIG. 10 is a diagram showing a contrast effect of target protein expression levels, and FIG. 11 shows the protein expression levels in a single cell. Both figures show that the yields of the real-time dynamic material supplementing operations and the manual timed material supplementing operation are comparable, with the yields of all dynamic material supplementing operations exceeding the yield of the manual material supplementing operation, where the dynamic material supplementing operations using methionine and glucose as markers is significantly better than the manual material supplementing operation.

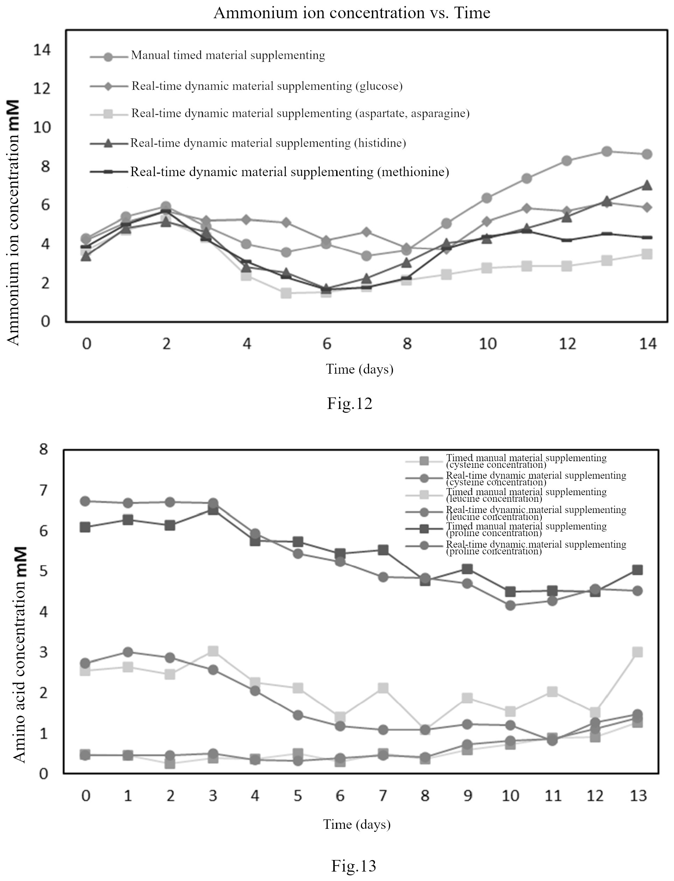

FIG. 12 is a diagram showing a contrast effect of ammonium ion concentration. The result indicates that in the real-time dynamic material supplementing operations, levels of ammonium ion that is a metabolic waste, are also controlled to be lower than that in the manual material supplementing operation. This performance can also increase the density and viability of viable cells, thereby ensuring high production of the target protein.

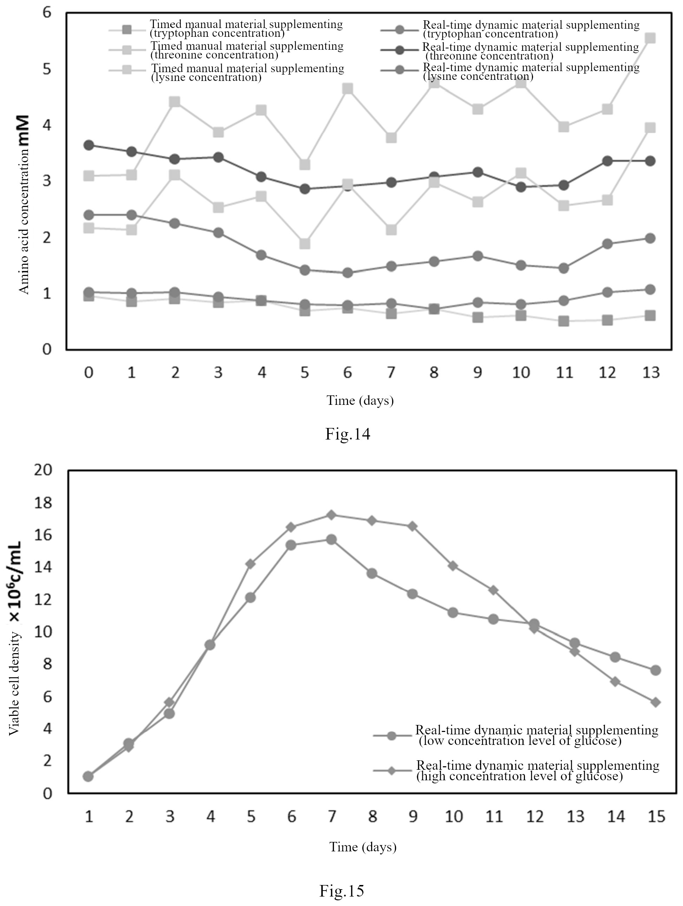

FIG. 13 is a diagram showing a contrast effect (I) of amino acid concentrations between the manual timed material supplementing and the real-time dynamic material supplementing when the marker is glucose, and FIG. 14 is a diagram showing a contrast effect (II) of amino acid concentrations between the manual timed material supplementing and the real-time dynamic material supplementing when the marker is glucose. The results show that different types of amino acids have different concentration levels, and the dynamic material supplementing has more stable concentration variation range and smaller variation amplitude than those of the manual material supplementing, which may bring a more stable nutritional environment, and is more suitable for cell growth and protein synthesis. In FIG. 13, the 5-6 mM level represents proline; the 2-4 mM level represents leucine; and the 0-2 mM level represents cysteine. In FIG. 14, the 3-6 mM level represents threonine; the 2-4 mM level represents lysine; and the 0-1 mM level represents tryptophan. The manual timed material supplementing is indicated by square marks; and the real-time dynamic material supplementing is indicated by circular marks.

In summary, after calculation and selecting, glucose is ultimately chosen as the marker in Case 1. Furthermore, in Case 1, there is a problem of lactate accumulation in the cultivation process. The selection of glucose as the marker can achieve not only the subsequent automatic material supplementing control, but also the control of the lactate level by controlling the concentration of glucose. In the traditional method, the supplemental amounts of material and glucose on the second or third day are regulated based on the data from daily offline measurement to control lactate. This method suffers from severe delays and significant fluctuations in glucose concentration in the cell culture solution (as shown in the circle-marked curve in FIG. 7). Although glucose levels are controlled, the long delays and fluctuating glucose concentration result in high lactate concentration, leading to low cell density and viability. The real-time dynamic material supplementing action in this embodiment effectively controls the glucose concentration within a low range throughout the culture process (as shown by the diamond-marked curve in FIG. 7), while maintaining the lactate level in a low concentration range at the end of the cell culture (as shown by the diamond-marked curve in FIG. 8), thereby maintaining a high cell viability (as shown by the diamond-marked curve in FIG. 6), and ultimately enabling the cells to synthesize more target proteins more efficiently at the end of the cell culture (FIG. 10). Meanwhile, the lactate dehydrogenase concentration curve also corroborates the phenomenon of low viability at the end of the culture (FIG. 9). The trends in the diagrams of the contrast effects of amino acids shown in FIGS. 13 and 14 also demonstrate that real-time dynamic material supplementing actions can reduce fluctuations in amino acid concentration in the cell culture solution when glucose is used as the marker for material supplementing, thus keeping the amino acid concentration within a stable concentration range.

In the comparative experiment, a combination of aspartic acid and asparagine, or histidine, or methionine is selected as the marker. In the cell culture process, asparagine and aspartic acid reach a dynamic equilibrium through acyl transfer, thus when selecting a marker, choosing only one of them will cause errors in the material supplementing operation, leading to failure of cell culture. Therefore, a sum of concentrations of these two substances in the cell culture solution is used as the marker to control the material supplementing operation. FIG. 12 shows ammonium ions as another toxic byproduct in the cell culture solution. However, by using amino acid concentration as the marker for feedback control to perform the real-time dynamic material supplementing, the concentration of ammonium ions in the cell culture solution is reduced by 100%. This phenomenon also helps improve cell viability and cell state, which in turn helps increasing the yield of target proteins. Meanwhile, the curves of amino acids control show that the performances of the other process parameters are similar.

### Embodiment 2

This embodiment examines the impact of different glucose concentration ranges on fermentation based on the automatic material supplementing strategy in Embodiment 1, so as to further verify the advantages of the automatic material supplementing strategy of the present disclosure.

The material supplementing operations at both high and low concentration levels are performed respectively by taking glucose as the marker and adopting the same real-time dynamic material supplementing action, and the measurement results of fermentation processes are shown below.

FIG. 15 is a diagram showing a contrast effect of viable cell density. The result indicates that the higher glucose concentration levels can maintain higher biomass (viable cell density levels).

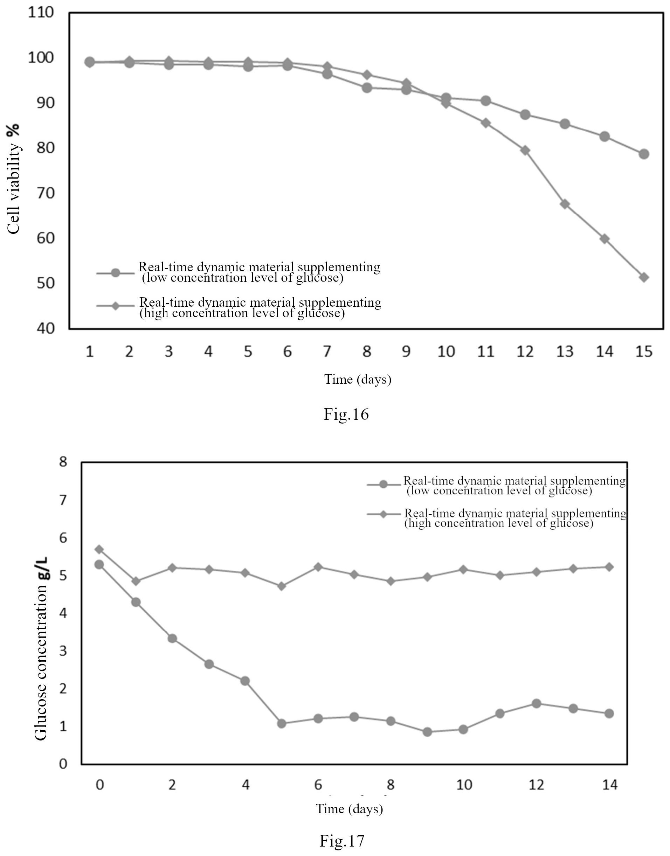

FIG. 16 is a diagram showing a contrast effect of cell viability. The result indicates that the higher glucose levels are less effective in maintaining viability in the later stage of cell culture, which may be because the higher glucose concentrations lead to the generation of more lactate.

FIG. 17 is a diagram showing a contrast effect of glucose concentration. The result indicates that, the diamond-shaped curve shows the higher glucose concentration, which is approximately 5-6 g/L.

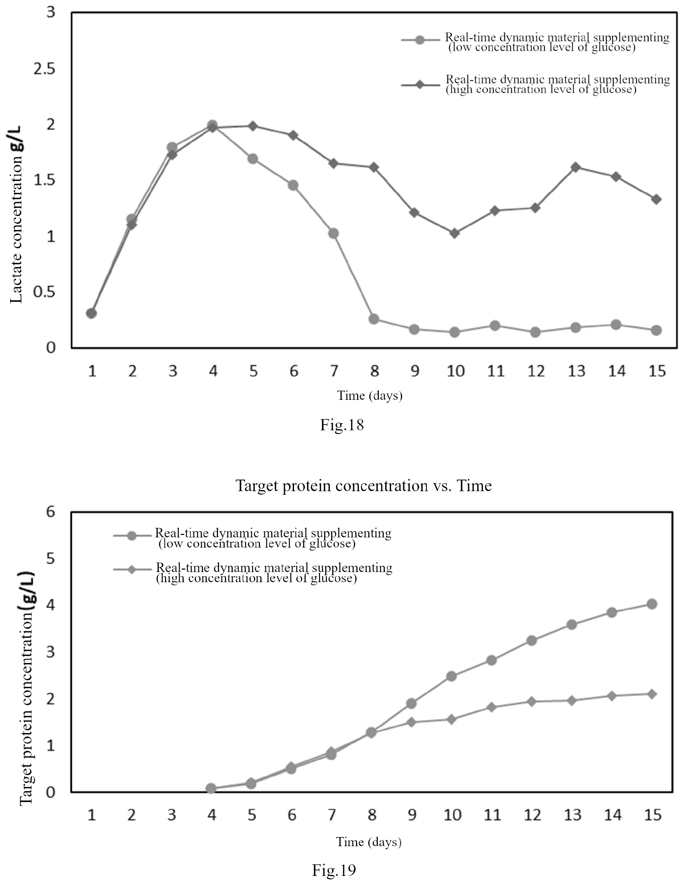

FIG. 18 is a diagram showing a contrast effect of lactate concentrations. The result indicates that the higher glucose concentration levels cannot maintain lower lactate levels. This may be because enrichment of glucose from the cellular glycolysis pathway leads to excessive lactate accumulation, while glucose does not enter the tricarboxylic acid cycle pathway.

FIG. 19 is a diagram showing a contrast effect of target protein concentration. The result indicates that excessively high lactate levels lead to excessively low pH in the culture solution. Cells in such low pH levels undergo changes in state, and protein synthesis pathways are hindered, resulting in lower yields.

In summary, in the case that glucose is selected as the marker for material supplementing, when different glucose concentration ranges are given and the same real-time dynamic material supplementing action is performed, it is also demonstrated that a lower level of glucose concentration can effectively control lactate concentration, thereby ensuring cell viability and stable yield. The above comparative experiments show that the automatic material supplementing strategy of the present disclosure can automatically generate a material supplementing strategy based on real-time measurement results, and the material supplementing strategy has better fermentation effects compared to the traditional material supplementing strategy.

### Embodiment 3

A control apparatus for cell culture material supplementing includes:
(1) A concentration of a component of a cell culture solution fed back by a process analysis tool is taken as a marker for feedback control.
   1.1 The process analysis tool includes but not limited to a Raman spectrometer, an infrared spectrometer, an online biochemical analyzer, an online high-performance liquid chromatograph, or an online mass spectrometer.
   1.2 The marker for feedback control is selected using the method of Case 1.
(2) Component concentration values are mapped to process value positions in a control module of a controller via a data transmission protocol (OPC/Modbus).
   2.1 The controller includes but not limited to Finesse, GE, or STR.
(3) Logic for processing component concentrations with multivariate analysis is loaded into the control module to calculate a supplemental amount. Process characteristics are identified to determine a time point for material supplementing.
   3.1 The control module for multivariate analysis and processing is developed based on the following software, including but not limited to SIMCA, R studio, or Matlab.
(4) A correlation formula between the supplemental amount and the time point for material supplementing is set.
(5) All operations are completed based on the supplemental amount and the time point for material supplementing.
(6) Alternatively, an amount of nutrients required by the cell at a moment is calculated based on the new component concentration value input at that moment, while the calculated value is mapped to the peristaltic pump of the controller.
(7) All operations are completed , and the specific effects are shown in FIGS. 4 to 19.

FIG. 20 is a schematic diagram of a control apparatus for cell culture material supplementing.

In summary, the present disclosure avoids the need for extensive sampling, reduces manual operation, and avoids human error by using the nutrient concentration in the cell culture solution to perform the feedback control of the material supplementing operation. Meanwhile, by real-time monitoring and automatic control, it is ensured that the nutrient levels in the cell culture solution are maintained as reasonable levels that meets the actual needs of the cells throughout the entire culture process.

The following describes in detail various embodiments of calculating the real-time measured concentrations of the components based on the real-time dynamic measurement results. It is understood that the following embodiments can be applied in both the case that the real-time measured concentration of each component for the historical time period is calculated based on the historical real-time dynamic measurement result, and the case that the real-time measured concentration of each component or the marker for the current time period is calculated based on the current real-time dynamic measurement result.

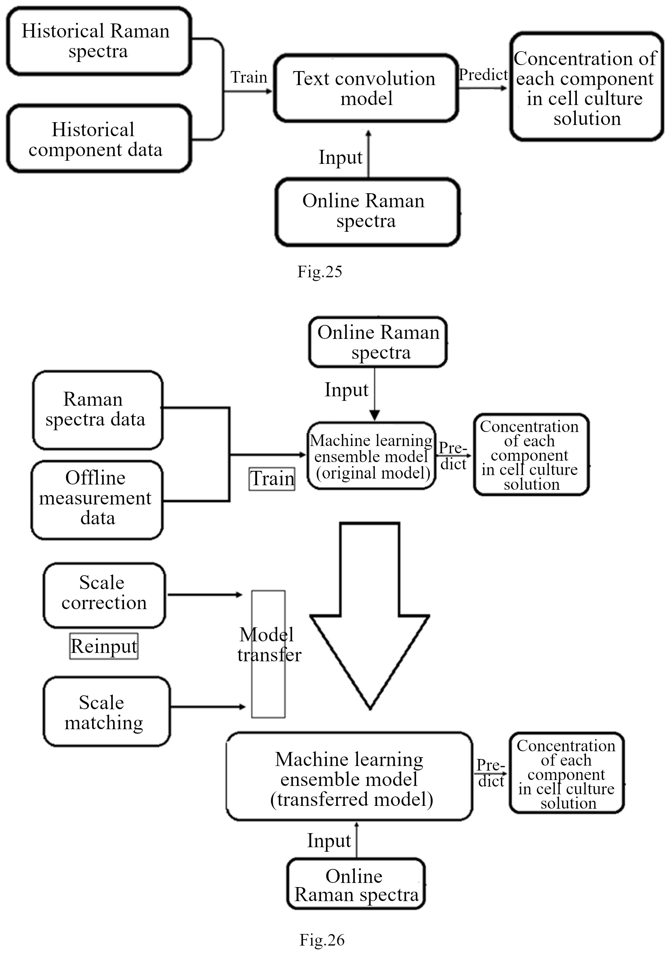

In some embodiments, the real-time dynamic measurement result is online spectral data (e.g., but not limited to, Raman spectral signal data, etc.), where calculating the real-time measured concentration of the component based on the real-time dynamic measurement result includes inputting the real-time dynamic measurement result into a trained text convolutional model to obtain the real-time measured concentration of the component. Specifically, the text convolution model is trained by taking the online spectral data of an indicator of a cell culture solution in a bioreactor at multiple different time points as samples and taking offline target values of the indicator measured by sampling the cell culture solution as labels. The text convolution model is configured to receive real-time measured spectral data of the indicator of a cell culture solution to be predicted and output a prediction value of the indicator. In some embodiments, the text convolution model is configured to: set a word vector mapping layer and perform feature dimensionality reduction; and obtain, using a fully connected structure, the prediction value of the indicator after averaging over a spatial dimension. In some embodiments, the text convolution model is configured to: further perform neighbor feature sampling after setting the word vector mapping layer and performing the feature dimensionality reduction. Specifically, the indicator may include one or more of: pH value, carbon dioxide partial pressure, sodium ion concentration, potassium ion concentration, glucose concentration, lactate concentration, ammonium ion concentration, glutamine concentration, glutamate concentration, target protein concentration, lactate dehydrogenase concentration, terbium ion concentration, phosphate ion concentration, osmotic pressure, viable cell density, cell viability, average diameter of viable cells, and amino acid concentration. For example, FIG. 25 schematically depicts a training process of the text convolution model based on historical Raman spectra and historical component data, as well as an application process of predicting the concentration of each component in the cell culture solution based on the input online Raman spectral data.

The text convolution is the application of Convolutional Neural Networks (CNNs) in text classification. The CNN is a deep learning method commonly used in image processing. When used for text processing, the CNN led to the development of a specialized text convolution model: the TextCNN model. Yoon Kim proposed TextCNN in his paper (2014 EMNLP), *Convolutional Neural Networks for Sentence Classification.* The core idea of CNNs is to capture local features. For text, the local features are sliding windows composed of several words, similar to N-grams. The advantage of CNNs is the ability to automatically combine and filter N-gram features to acquire semantic information at different levels of abstraction. The CNN is applied in text classification tasks to utilize multiple convolutional kernels of different sizes to extract key information from sentences, thereby enabling better capture of local relevance.

This property makes the CNN well applicable to the situation described in the present disclosure, because the various indicators of the culture solution in the present disclosure are often interrelated. For example, an increase in carbon dioxide partial pressure may lead to a decrease in pH value, while also increasing an overall pressure of the system. Such parametric properties are similar to those of natural language processed by CNNs.

The present disclosure allows the use of spectral data as measurement data. In some examples of the present disclosure, the Raman spectrometer is used to measure Raman spectral signals of the cell culture solution in real time during the cell culture as the measurement data (training set), and the machine learning model is used to analyze the Raman spectral data in real time and establish a relation model between the Raman spectral data and the various indicators of the culture solution measured offline at corresponding time points.

The Raman spectrum is a vibrational spectrum that detects and identifies molecules of substance by detecting the Raman spectrum generated by the Raman scattering effect on a sample under measurement in response to excitation light, which enables non-destructive analysis of chemical compositions and molecular structures. The number, frequency shift, intensity and shape of the Raman spectral bands generated by the Raman scattering effect of the substance are directly related to the vibration and rotation of the molecules of the substance. In particular, under certain conditions, the intensity of the Raman spectrum is linearly related to the concentration of the substance, which allows for the measurement of the structure, composition, and concentration of the substance. Compared with other spectroscopic analysis methods such as infrared, near-infrared, and ultraviolet fluorescence, Raman spectroscopy has significant advantages including: a wide measurement range; non-destructive, rapid, and pollution-free operation; long-distance measurement technology; and high measurement sensitivity.

Therefore, with the improvement of laser sampling and detector technology, the application of Raman spectroscopy in polymer, pharmaceutical, biomanufacturing and biomedical analysis has surged in the past three decades. Thanks to these technological advancements, Raman spectroscopy has now become a practical analytical technology used both in and out of laboratories. In the field of bioreactor pharmaceuticals, Raman spectroscopy is often used for online monitoring. Since the first reported application of in-situ Raman measurement in biomanufacturing, Raman spectroscopy has been used to provide online real-time predictions of several key process states such as glucose, lactate, glutamate, glutamine, ammonia, and VCD.

However, according to the principles and characteristics of Raman spectroscopy, a certain value or certain values in certain bands are very sensitive to the changes in component concentration and have a significant impact on the prediction results. Changes of some floating-point values are relatively subtle and need to be expanded and mapped into a two-dimensional spatial structure to facilitate extraction thereof. Furthermore, the affected bands may be extensive, and the extent is not fixed. Therefore, convolutional neural networks with fixed convolutional kernels often fail to accurately capture specific features.

Based on the above reasons, the inventors of the present disclosure refer to the model structure of TextCNN, which is frequently used in text processing. One-dimensional convolutions with different kernel sizes are used to extract features from neighborhoods of different step lengths in the mapped feature map, and the spatial correlation constraints of the intermediate feature extraction layer is used to guide the fully connected operation to preserve the feature relationships of original positional neighbors, so as to accurately predict indicators of components.

In the present disclosure, the concentration, pressure, density, temperature, content, and other items in the bioreactor are collectively referred to as indicators.

The following specific embodiments further illustrate the method using text convolution models.

### Embodiment 4

First, Raman spectral data and offline target values are collected. A Raman spectrometer is used to obtain online spectral data of different indicators changing over time from a cell culture solution in a bioreactor, and offline target values of the indicators are calculated based on time. At the same time, samples are taken at regular intervals during the experiment, and an offline measurement device is used to obtain offline target values of each batch to correct and calculate the offline target values.

Specifically, a cell line that produces monoclonal antibodies (Chinese hamster ovary cells) is used in the cell culture. The culture conditions for this cell line in the shaker (Kuhner) for the seed phase are as follows: 36.5°C, a rotational speed of 110 rpm, a CO₂ concentration level of 6%, and Cytiva's Hyclone Actipro as a culture medium. The culture conditions for this cell line in the production culture phase are as follows: 3L and 250L reactors with initial culture volumes of 1.5L and 140L respectively, a culture temperature of 36.5°C, a pH set value of 6.90±0.25, a dissolved oxygen saturation of 40%, an initial inoculation density of 1.0×10⁶ cells/mL, and also Cytiva's Hyclone Cell Boost 7a/7b (10%/1%) as a supplemental culture medium.

The online spectral data means full Raman shift spectral data collected by the Raman spectrometer. In the present disclosure, a Raman Rxn2 analyzer equipped with an immersed optical probe (Kaiser Optical Systems) is used. The probe is installed in the 3L bioreactor (Applikon) and directly immersed in the cell culture suspension. Raman spectra of different bioreactors are recorded throughout the experiment process. For a single recorded spectrum, 30 subsequent spectra are captured with 10-second exposure time and averaged, resulting in an acquisition interval of approximately 5 minutes between acquisition operations on the bioreactor. The laser excitation wavelength is 785 nm, providing spectral coverage (Raman shift) of 100-3425 cm⁻¹.

The offline target values mean cell culture solution indicators obtained from real-time on-site sample measurement. In the production culture phase, the probe of the Raman device is placed into the culture solution. Samples are taken five times daily, and the viable cell density is measured using Beckman's Vi-Cell XR; glucose concentration, lactate concentration, and target protein concentration are measured using Roche's Cedex Bio analyzer; and amino acid concentrations are measured using Agilent's HPLC.

Secondly, a machine learning model is built. The process of building the machine learning model includes: data processing, model building, data parameter tuning, and model validation.

In the data processing: spectral data is read, each sample contains a spectral file and an offline target value; the spectral file is converted into spectral values, and the measurement time of the spectrum and the measurement time of the offline target value are matched one-to-one to acquire the feature data (spectral values corresponding to different Raman shifts) and target values for modeling. Data preprocessing involves random splitting of training data, validation data, and test data, standardizing and normalizing of the feature values of the training data, and standardizing of the test data according to the standardization rules of the training data.

In the model building: spectral data of different sizes and lengths is used as input, and multiple machine learning models are built. The machine learning models built in this embodiment include Partial Least Squares Regression (PLS), XgBoost, Convolutional Neural Network (CNN), Residual Network (ResNet), and Text Convolutional (TextCNN) models.

In the data parameter tuning: the model is trained using the training set, and hyperparameters of the model are turned based on performance on the validation set. The specific parameters for the respective models are as follows.

PLS -- after filtering with a Savitzky-Golay filter, a second derivative is calculated and input into the model, and n_components is 5.

Xgboost -- the spectral data is normalized first, then the dimensionality is reduced to 50 by Principal Component Analysis (PCA), and n_components is set as 500.

Convolutional Neural Network (CNN) model -- one-dimensional convolution is superimposed with a max-pooling layer, which is repeated for 4 times, then two fully connected layers are connected to output the result.

Residual Neural Network (ResNet) -- at least two residual network layers are included, each residual network layer containing multiple one-dimensional convolutional layers, multiple max-pooling layers, and a fully connected layer.

Text Convolution (TextCNN) -- when building the text convolution model, the following means are further employed.
1) Setting a word vector mapping layer: the input one-dimensional floating-point vector is mapped to be a two-dimensional vector with specified length and feature dimension. Since each Raman shift value has only one floating-point number in the mapping, the word vector matrix cannot be used when performing feature extraction because the magnitude of the specific numerical value needs to be considered. Therefore, the fully connected structure is used here to complete the feature mapping, so as to establish a fixed mapping relationship between the input and the output. At the same time, the spatial position structure is preserved to a certain extent while completing the feature mapping.
2) Performing feature dimensionality reduction: when directly mapping the input spectrum values of which the floating-point vector has a length of 3000, a feature vector of 3000×N (N is the feature mapping dimension) will be generated, so it is necessary to compress the dimension generated after mapping, so as to prevent the introduction of a large amount of computational consumption in the early stage of computations. In the specific implementation, the feature dimension after mapping is predefined as A×N, where A is the length of the mapped feature vector and N is the feature dimension. The original input vector is directly mapped to be a vector with a length of A×N through the fully connected structure, and then the reshape operation is performed to generate the mapped features, thus compressing the feature length while mapping the features.
3) Neighbor feature sampling: if necessary, after setting the word vector mapping layer and performing feature dimensionality reduction, neighbor feature sampling may be further performed. Different substances are known to correspond to one or more feature regions in the spectrum, so for feature extraction and calculation, not feature extraction along all the input dimensions are effective, and effective feature extraction requires the ability to perform feature calculation between adjacent spectra. Since the fully connected operation, which integrates global features, disrupts the original spatial structure distribution during the setting of word vector mapping layers and performing of feature dimensionality reduction, it is necessary to reintroduce spatial relationships in subsequent structures. Specifically, referring to the textCNN structure, one-dimensional convolutions with different kernel sizes are used to extract features from neighborhoods of different step lengths in the mapped feature map. Spatial correlation constraints of the intermediate feature extraction layer are used to guide the fully connected operation to preserve the feature relationships of original positional neighbors.
4) After averaging over the spatial dimension for prediction, the fully connected structure is used to predict the corresponding target value.

First, validation results of the model on the test set are obtained. In addition, to ensure the production usability of the models, prospective wet experiments are selected to evaluate the performances of all models.

Specifically, the flow of the wet experiment is described as follows: under the same culture conditions as the historical training data, two Raman sensors are used to scan two bioreactors at regular intervals to collect Raman spectral datal; data of four time points is collected per day, and samples are collected at the same time to collect offline data; and a total of 140 pieces of data are collected for one complete culture cycle. Root-mean-square error (RMSE) values of offline and online data are calculated, and results are shown in Table 2. After comparing multiple models with various parameters, it is found that TextCNN has a very significant advantage for most indicators.

**Table 2. RMSE of model predicted values and offline measured values in the cell culture solution**

| Process parameters | CNN | Resnet | Xgboost | PLS | TextCNN | Unit | Parameter range |
|---|---|---|---|---|---|---|---|
| viable cell density (VCD) | 5.69 | 5.36 | 4.68 | 5.00 | 2.91 | E6cell/mL | 0.70-49.00 |
| Glucose | 1.51 | 1.69 | 1.32 | 1.02 | 1.37 | g/L | 0.50-9.00 |
| Terbium ion (Iron) | 0.36 | 0.39 | 0.29 | 0.26 | 0.18 | mg/L | 0.85-1.80 |
| Glutamine | 0.76 | 0.94 | 0.93 | 0.68 | 0.57 | mM | 0.45-4.00 |
| pH | 0.21 | 0.50 | 0.19 | 0.18 | 0.15 | | 6.58-7.27 |
| Carbon dioxide partial pressure (pCO₂) | 10.81 | 8.77 | 9.53 | 12.23 | 8.37 | mmHg | 12.1-71.9 |
| Sodium ion (Na⁺) | 8.73 | 7.67 | 14.64 | 11.44 | 9.24 | mM | 88-133 |
| Potassium ion (K⁺) | 1.53 | 1.30 | 0.94 | 0.62 | 1.27 | mM | 3.85-18.10 |
| Lactate | 0.64 | 0.62 | 0.80 | 0.43 | 0.50 | g/L | 0.15-2.45 |
| Ammonium ion (NH4⁺) | 1.55 | 1.46 | 1.18 | 1.08 | 0.89 | mM | 0.45-9.00 |
| Glutamate | 3.13 | 3.41 | 2.32 | 0.39 | 0.78 | mM | 2.70-15.00 |
| Osmotic pressure (OSMO) | 35.45 | 36.28 | 24.27 | 32.47 | 23.49 | mO̅sm/kg | 260-450 |
| Cell viability (Viability) | 20.77 | 19.22 | 17.45 | 10.88 | 16.78 | % | 25-99.9 |
| average diameter of viable cells (AvgDiam) | 1.91 | 1.63 | 1.97 | 1.69 | 1.51 | um | 11.50-19.50 |
| Target protein (Titer) | 0.17 | 0.83 | 1.08 | 1.76 | 0.23 | g/L | 0-6.0 |
| Lactate dehydrogenase (LDH) | 1062.71 | 587.37 | 1355.90 | 272.70 | 77.22 | U/L | 0-6000 |
| Phosphate ion (Phosphate) | 212.52 | 244.77 | 306.57 | 189.49 | 250.93 | mg/L | 350-1200 |

In some other embodiments, calculating the real-time measured concentration of the component based on the real-time dynamic measurement result includes inputting the real-time dynamic measurement result into a machine learning ensemble model for predicting a component concentration in a cell culture solution to obtain the real-time measured concentration of the component. The machine learning ensemble model is built by: obtaining a dataset of a component concentration of a cell culture solution in a bioreactor, the dataset including a training dataset, a validation dataset, and a test dataset; building multiple single prediction models by using multiple machine learning algorithms, respectively, where each of the multiple single prediction models performs prediction on the test dataset after being trained with the training dataset and validated with the validation dataset; obtaining a square sum of prediction errors of the multiple single prediction models by comparing prediction results of the multiple single prediction models with the test dataset, and determining weights of the multiple single prediction models in the machine learning ensemble model based on a magnitude of the square sum of the prediction errors; and obtaining the machine learning ensemble model by combining the multiple single prediction models through a weight assignment method.

In some other embodiments, calculating the real-time measured concentration of the component based on the real-time dynamic measurement result includes inputting the real-time dynamic measurement result into a machine learning ensemble model for predicting a component concentration in a cell culture solution to obtain the real-time measured concentration of the component. Unlike the previous embodiment, the machine learning ensemble model here is a transferred model obtained by performing model transfer relative to an original model. The original model here may be, for example, the machine learning ensemble model of the previous embodiment, which is specifically built by: obtaining a dataset of a component concentration of a cell culture solution in a bioreactor, the dataset including a training dataset, a validation dataset, and a test dataset; building multiple single prediction models by using multiple machine learning algorithms, respectively, where each of the multiple single prediction models performs prediction on the test dataset after being trained with the training dataset and validated with the validation dataset; obtaining a square sum of prediction errors of the multiple single prediction models by comparing prediction results of the multiple single prediction models with the test dataset, and determining weights of the multiple single prediction models in the original model based on a magnitude of the square sum of the prediction errors; and obtaining the original model by combining the multiple single prediction models through a weight assignment method. Furthermore, the model transfer includes: obtaining an original dataset used for building the original model, and a new-batch training dataset of the component concentration of a cell culture solution of a new batch of biological reactions, the original dataset comprising an original training dataset, an original validation dataset, and an original test dataset; obtaining a new training dataset by performing scale correction or scale matching on the new-batch training dataset and the original training dataset; building multiple single prediction models by using multiple machine learning algorithms, respectively, with the new training dataset, the original validation dataset, and the original test dataset, where each of the multiple single prediction models performs prediction on the original test dataset after being trained with the new training dataset and validated with the original validation dataset; obtaining a square sum of prediction errors of the multiple single prediction models by comparing prediction results of the multiple single prediction models with the original test dataset, and determining weights of the multiple single prediction models in the transferred model based on a magnitude of the square sum of the prediction errors; and obtaining the transferred model by combining the multiple single prediction models through a weight assignment method.

Specifically, the scaling correction may include incorporating a specified proportion of data from the new-batch training dataset into the original training dataset used for building the original model, and the scaling matching may include incorporating new-batch training data, with a numerical difference from original training data collected at an identical time point as the new-batch training data in the original training dataset being less than a specified threshold, from the new-batch training dataset into the original training dataset. In some examples, the specified proportion is a value selected from 1% to 10%, or a value selected from 1.5% to 7.5%, or a value selected from 2% to 5%. In some examples, the specified threshold is a value selected from 1% to 10%, or a value selected from 3% to 8%, or a value selected from 4% to 6%, or 5%.

For example, the multiple machine learning algorithms may include at least two selected from: Partial Least Squares, Cubist, Random Forest, Support Vector Machine, or Time Series. The dataset of the component concentration may include online Raman spectral data and corresponding offline measurement data thereof, and a sampling time point of the offline measurement data is matched with corresponding online Raman spectral data. As a non-limiting example, the component concentration may be selected from: viable cell density, glucose concentration, lactate concentration, target product concentration, and amino acid concentration . In some examples, data preprocessing may further be performed on the Raman spectral data, the data preprocessing including at least one of: screening out abnormal data points, removing spikes, Raman shift correction and light intensity correction, baseline calibration, smoothing, or differentiation.

For example, FIG. 26 schematically depicts a training process of the machine learning ensemble model as the original model based on the Raman spectral data and the offline measurement data, as well as an application process of predicting the concentration of each component in the cell culture solution based on the input online Raman spectral data. FIG. 26 also schematically depicts a training process of the machine learning ensemble model as the transferred model based on the scale correction or the scale matching, as well as an application process of predicting the concentration of each component in the cell culture solution based on the input online Raman spectral data. The flow shown in FIG. 26 may be divided into three main steps: data preparation, model building, and validation and tuning. In addition, considering the model transfer, the present disclosure further includes steps of data correction and data matching. The following are examples showing these steps.

### (1) Data preparation

In the embodiments of the present disclosure, although other methods that can detect the concentration of each component in the cell culture solution and meet the requirements of the present disclosure may also be used, the Raman spectral signal measured by the Raman spectrometer from the cell culture solution is mainly used as measurement data (training set) and validation data (validation set).

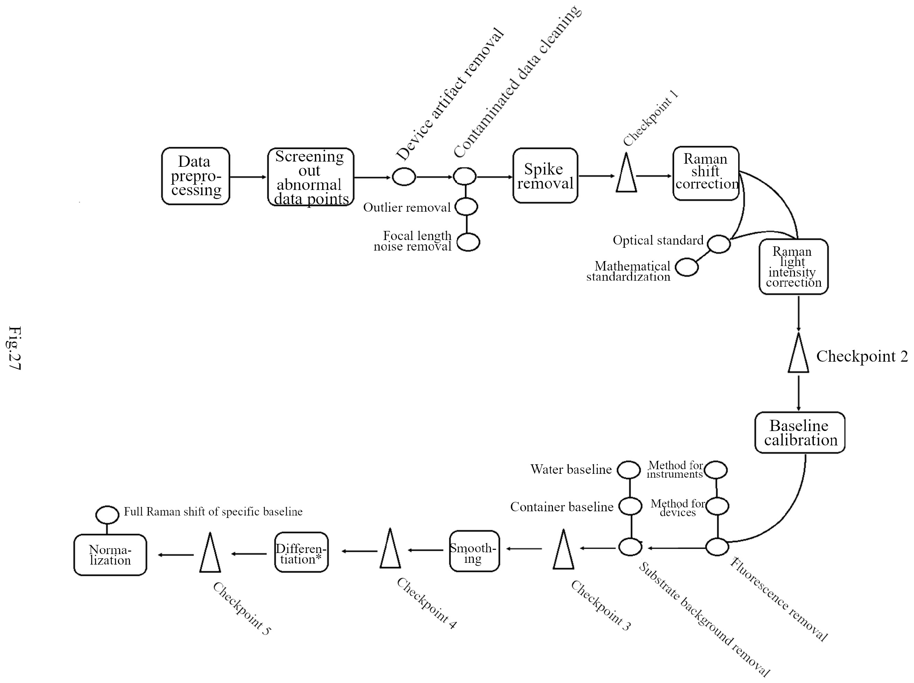

In the embodiments of the present disclosure, after obtaining Raman spectral measurement data, the data is first preprocessed to be better applied in machine learning models. Referring to FIG. 27, in the embodiments of the present disclosure, the data is further processed by the flow shown in FIG. 27.

When entering the data preprocessing flow, the following steps are performed for data preprocessing.
(a) Screening out abnormal data points: the abnormal data points are initially screened out using simple statistical methods. Specifically, abnormal data that deviates significantly from the main data may be removed by calculating the mean, median, standard deviation, etc.
(b) Spike removal: cosmic spikes in Raman spectra originate from electrons generated by high-energy cosmic particles hitting the CCD or complementary metal-oxide-semiconductor detectors, and appear randomly in the Raman spectra, exhibiting very narrow but extremely strong spectral features. Due to such high intensity, the spike makes data analysis difficult. If interfering spikes exist, the results of standardization and feature extraction are meaningless. Once a spike is detected, it can be removed by linear interpolation based on two boundary points of the spike. Alternatively, the spike may be replaced by continuous measured values taken at the same wavenumber position as the spike. In this case, the fluorescence difference and intensity change between two measurements must be taken into account.
(c) Raman shift correction and light intensity correction: Raman spectroscopy should produce the same results in any measurement environment, device or other conditions, but this is not the case in practice. Conversely, under varying measurement conditions and the like, spectral changes are observed between instruments over time. A well-designed standardization method is needed to eliminate these unwanted spectral variations and to standardize all measured Raman spectra relative to the same reference. One of the most fundamental methods of this standardization for Raman spectroscopy is spectrometer correction, which includes wavenumber and intensity correction. It is an ideal method for unifying Raman spectra to use a stable and identical spectral corrector to correct the shift and intensity, and then to verify the results with the same optical standards. In addition to the correction, the wavenumber axis is calibrated by fitting a (polynomial) function between measured and theoretical positions of well-defined Raman bands of a wavenumber standard. The intensity axis is calibrated by dividing measured Raman intensities by the instrument's intensity response function, which is derived as a ratio between measured and theoretical emissions of an intensity standard within a wavenumber range of interest.
(d) Baseline calibration: baseline calibration has two meanings, namely, removing a spectrum of substrate information or removing a fluorescence baseline. The former is used to remove a Raman signal of a substrate from the measured Raman spectrum; and the latter aims to remove a fluorescence of a sample expressed as a slowly changing baseline in the Raman spectrum. If the substrate has a large number of Raman bands, especially if these Raman bands overlap with the sample's Raman bands, then the substrate's contribution needs to be removed. Therefore, the spectrum of the substrate is usually needed as the reference to estimate the contribution of the substrate in the recorded Raman spectrum. For heterogeneous substrates, statistical methods may be useful; for example, multivariate curve resolution may be used to handle the contributions of such heterogeneous substrates. Fluorescence baseline removal is often more complex than substrate calibration because the fluorescence baseline depends on the sample and setup. Such fluorescence baselines are mostly removed using mathematical methods, such as calculating derivative spectra, sensitive nonlinear iterative peak shearing algorithms, asymmetric least squares (ALS) smoothing, modified polynomial fitting, standard normal variables, multiplicative scattering calibration, and extended multiplicative signal calibration (EMSC). These methods are flexible, easy to use, require no instrument modification, and have adequate performances in most cases. However, if the fluorescence intensity is too strong for mathematical calibration, an instrument-modification-based approach may be required. This category of technologies includes time-series Raman spectroscopy, modulation Raman spectroscopy, and displacement-excited Raman differential spectroscopy.
(e) Smoothing: in Raman spectroscopy analysis, smoothing or filtering may be selected, which may be accomplished through spectral and/or spatial filtering. The spectral filtering is performed to remove noise along the wavenumber axis using a low-pass filter. The filter may be average, median, Gaussian, polynomial function filters, etc. The spatial filtering shares a similar concept with the spectral filtering, but it applies the low-pass filter to a spatial domain. Both methods have their advantages and disadvantages. The spectral filtering reduces spectral resolution but preserves spatial resolution, and vice versa.
(f) Differentiation: the differentiation is optional and its main purpose is to further improve signal-to-noise ratio values.
(g) Normalization: the normalization aims to eliminate effects of excitation intensity fluctuations or focus changes, which may be performed using conventional normalization methods.

As shown in FIG. 27, after steps of (b) spike removal, (c) Raman shift correction and light intensity correction, (d) baseline calibration, (e) smoothing and optional (f) differentiation, checkpoints are set to check the data preprocessing effect of the previous step to determine whether the previous step has been over-processed. If over-processing is identified, the flow turns back to the previous step, which is then adjusted and performed again.

Specifically, at checkpoint 1, it is determined whether the spikes have been excessively removed. At checkpoint 2, it is determined whether Raman shift and light intensity drift still exist. At checkpoint 3, it is determined whether the characteristic spectra of the fluorescence and the substrate still exist. At checkpoint 4, it is determined whether there is any loss of displacement and intensity after smoothing. At checkpoint 5, it is determined whether the original spectrum is distorted after differentiation. Based on the determination results of respective checkpoints, since differentiation is not required for every preprocessing operation, the determination is not performed at the checkpoint 5 if differentiation is not performed.

### (2) Model building

Typically, after obtaining data measured by the Raman spectroscopy, technicians attempt to convert Raman signals into digital information and identifiable corresponding data, and further identify a substance based on a similarity between the measured spectrum and the spectrum of known substances in a spectral database, such as identifying a concentration/content of each component in a cell culture solution in the present disclosure. However, this is not feasible in the case of Raman spectroscopy measurement on cell culture processes in bioreactors as in the present disclosure, and the reason is as follows: typically, technicians can identify the measured substances and concentrations/contents thereof by comparing the measured Raman spectral data with data from the database of known substances; however, the cell culture is a very complex process that may produce a wide variety of substances, and the measurable sample components in the cell culture solution are too complex. In particular, when the measured spectrum contains signals from the substrate, the model prediction will be biased towards the substrate, meaning that the measurement results largely reflect the concentrations/contents of components of the culture medium and/or the substrate that make up the majority of the mass of the cell culture solution, rather than the concentrations/contents of certain characteristic components that affect the cell culture process.

Therefore, in the embodiments of the present disclosure, the machine learning-based method is used to extract characteristic spectra according to algorithms and assign offline measurement values to the characteristic spectra to obtain resolved measurement spectra.

In the embodiments of the present disclosure, for the preprocessed data, five algorithms, i.e., Partial Least Squares (PLS), Cubist, Random Forest (RF), Support Vector Machine (SVM), and Time Series, may be used, for example, to build single prediction models, respectively. Then, prediction results are processed by the Reciprocal Variance (RV) method to determine the weight of each algorithm in the ensemble model, and the new prediction result is calculated to build the machine learning ensemble model.

The example algorithms used in the present disclosure are respectively described below.

### Partial Least Squares (PLS):

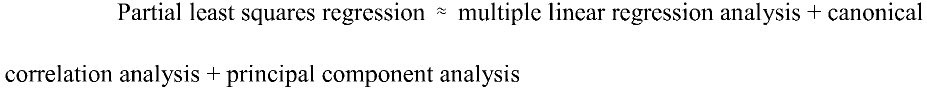

Partial least squares regression ≈ multiple linear regression analysis + canonical correlation analysis + principal component analysis

Step 1: original data X and Y are centralized to acquire X0 and Y0. A column of Y0, generally the column with the largest variance, is selected as u1. The formula for the sample covariance of standardized data is: $cov \left(X, Y\right) = \frac{{X}^{T} Y}{n - 1}$

Step 2: transformation weights (w1, c1) and factors (u1, t1) of X and Y are iteratively solved until convergence. The information u1 of Y is used to find the transformation weight w1 of X (w1 realizes a transformation from X0 to factor t1, t1=X0*w1) and factor t1, so that the information of X0 can be approximatively expressed by t1. ${w}_{1} = {X}_{0}^{T} {u}_{1} / {u}_{1}^{t} {u}_{1}$ $\left‖{w}_{1}\right‖ \to 1$ ${t}_{1} = {X}_{0} {w}_{1}$

The information t1 of X is used to find the transformation weight c1 of Y (c1 realizes a transformation from Y0 to a factor u1, u1=Y*c1), and factor u1 is updated, so that the information of Y0 may be approximatively expressed by t1. ${c}_{1} = {Y}_{0}^{T} {t}_{1} / {t}_{1}^{t} {t}_{1}$ $\left‖{c}_{1}\right‖ \to 1$ ${u}_{1}^{∗} = {Y}_{0} {c}_{1}$

It is determined whether a reasonable solution has been found. If Δu < threshold (e.g., 10⁻⁶), it continues to perform the following steps; otherwise, it is taken that ul=ul * and the flow turns back to step 2. ${u}_{Δ} = {u}_{1}^{*} - {u}_{1}$ $Δu = {u}_{Δ}^{T} {u}_{Δ}$

Step 3: residual matrices of X and Y are calculated, and a loading p1 of X (p1 reflects the direct relationship between X0 and factor t1) is calculated. ${X}_{0} = {t}_{1} {p}_{1}^{T}$ ${p}_{1} = {X}_{0}^{T} {t}_{1} / {t}_{1}^{T} {t}_{1}$

Step 4: X1 and Y1 are used to repeat the above steps to solve for a next batch of PLS parameters.

### Cubist:

An ensemble learning algorithm based on a model tree is selected for the Cubist model. When a first model tree is built following the M5 model tree rules, a next model tree is an adapted version of a result of a training set. If the model overestimates a target value, the next model responds by regulating downwards, and so on. The final estimated value is an average of model calculation values of respective model trees.

What's on a node of the model tree is not a constant, but rather a linear function model. The standard for segmenting space is not to reduce the square error, but to reduce the standard deviation of samples.

### M5 Model Tree:

A standard deviation of Y values (i.e., target attribute values) of the samples covered by one node is regarded as a measure of error. $SDR = sd \left(T\right) - \sum i \frac{\left|{T}_{i}\right|}{\left|T\right|} sd \left({T}_{i}\right)$

T is a set of instances that reach this node, |T| represents a size of the set, sd represents the standard deviation, and Ti is a set of instances in an i^{th} subtree.

Node pruning of the model tree is a bottom-up recursive process. A linear regression method is used to fit a regression equation for each node, and a root mean square error of predictions of the regression function is calculated. ${R}_{MSE} = \sqrt{\frac{1}{n} {\sum }_{i}^{n} {y}_{i} - R {\left(β, {x}_{i}\right)}^{2}}$

A decrease in MSE from each node to its child node is calculated. ${E}_{R} = \left|N\right| {R}_{MSE} - \sum i \left|{N}_{i}\right| {R}_{MSEi}$

### Random Forest (RF):

The random forest model is a comprehensive learning model that uses the Bagging algorithm to build multiple decision tree models and calculates an average value of results of all decision tree models.

### Enhancements in the Bagging Algorithm:

${F}_{n} \left(x\right) = {F}_{M - 1} \left(x\right) + {argmin}_{h} {\sum }_{i = 1}^{n} L \left({y}_{i},{F}_{M - 1}\left({x}_{i}\right)+h\left({x}_{i}\right)\right)$

### Support Vector Machine (SVM):

The support vector machine is a method for classifying linear and nonlinear data.

### Linearly separable support vector machine:

W is a weight vector, b is a bias scalar, and a training objective formula when a parameter of the training objective data is minimized is as follows: $w = arg max \frac{2}{\left‖w\right‖}$ $w = arg min \frac{{\left‖w\right‖}^{2}}{2}$

If a constraint of the above formula is expressed as: ${∀}_{i} {y}_{i} \left({wx}_{i}+b\right) \geq 1 ,$
then the problem of separating hyperplanes with the largest interval is expressed as follows: $min \frac{\left‖{x}^{2}\right‖}{2} s . t . ∀ {iy}_{i} \left({wx}_{i}+b\right) \geq 1$

Nonlinear separable support vector machine:
the kernel function method is introduced.

The polynomial kernel function is as follows: $K \left({x}_{i}, {y}_{j}\right) = {\left({x}_{i}^{T}{x}_{j}\right)}^{d}$

The Gaussian kernel function is as follows: $K \left({x}_{i}, {y}_{j}\right) = exp \left(-\frac{{\left‖{x}_{i}-{x}_{j}\right‖}^{2}}{2 {σ}^{2}}\right)$

### Time series exponent:

The exponential smoothing method is used to make predictions and determinations by calculating exponential smoothed values and combining them with the time series prediction model. The principle is that the exponential smoothed value of any value is a weighted average of its actual value and a previous value.

When the time series shows a clear trend change, the single exponential smoothing method is used for prediction. ${S}_{t + 1}^{1} = {αX}_{t} + \left(1-α\right) {S}_{t}^{1}$

The double exponential smoothing is a further smoothing operation of the single exponential smoothing and is suitable for time series with linear trends. ${S}_{t}^{1} = {αX}_{t} + \left(1-α\right) {S}_{t - 1}^{1}$ ${S}_{t}^{2} = α {S}_{t}^{1} + \left(1-α\right) {S}_{t - 1}^{2}$

A formula for predicting future T cycles is as follows: ${y}_{t + T} = {A}_{t} + {B}_{t} T$ ${A}_{t} = 2 {S}_{t}^{1} + {S}_{t}^{2}$ ${B}_{t} = \left(\frac{a}{1 - a}\right) \left({S}_{t}^{1}+{S}_{t}^{2}\right)$

### Reciprocal Variance method:

The results of the above model algorithms are combined, and weights of respective algorithms in the ensemble model are determined based on a magnitude of a square sum of prediction errors using the reciprocal variance method: ${w}_{i} = \frac{{Q}_{i}^{- 1}}{{\sum }_{i = 1}^{m} {Q}_{i}^{- 1}} ,$
where Qi is a square sum of differences between actual values and predicted values. ${Q}_{i} = {\sum }_{t = 1}^{n} {\left({y}_{t}-{y}_{t}^{′}\left(i\right)\right)}^{2}$

Five prediction models are built using the above modeling processes, and then the five models are combined into a new machine learning ensemble model using a weight assignment method.

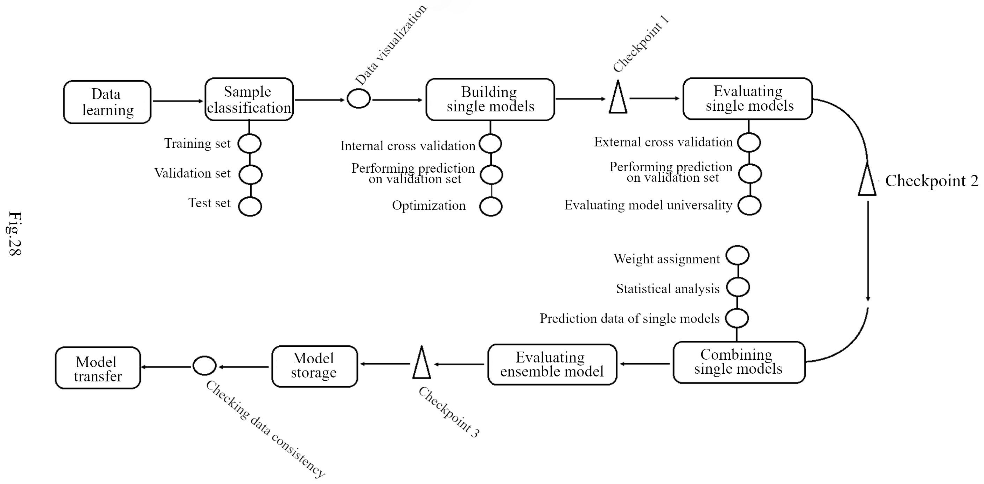

In the present disclosure, a workflow for data learning is further established, as shown in FIG. 28, which illustrates the flow of building models and comparing predictive performances of respective models for selection.

The data learning workflow includes at least the following steps:
(I) Sample classification: in a first stage of data learning, statistical sampling is carried out to prepare data for statistical modeling. An effective statistical model with a limited sample size is achieved. An accessible dataset is divided into three subsets: training, validation, and test datasets. The three subsets are used for model training, model optimization, and model evaluation, respectively. In many cases, data splitting is repeated multiple times, such as in cross-validation (CV) or guiding methods. Differently separated three subsets are generated repeatedly for each time, and statistical modeling is performed multiple times. This operation provides an opportunity to verify the stability and reproducibility of the model, for example, based on the mean and standard deviation of the accuracies or the root mean square error (RMSE) calculated from multiple predictions on different test datasets.
(II) Building of single prediction models: statistical modeling and machine learning start with reducing dimensionality. This is particularly important for Raman spectroscopy, where the dataset includes a large number of relevant features and the sample size is limited. The dimensionality reduction has two main benefits. Firstly, it makes visualization simpler and clearer, thus helping to better outline the features of the dataset; secondly, it can improve and accelerate subsequent modeling by removing redundant information and extracting useful features from the data. In a second stage of data learning, an output of the dimensionality reduction is input into subsequent models, which may be clustering, classification, or regression models. Models that perform such operation may be categorized as linear or nonlinear, parametric or nonparametric, supervised or unsupervised models. While the choice of model to be used is data-dependent, it should be remembered that the universality of a model is likely to decrease as its complexity increases. The model should be as simple as possible without sacrificing performance, which means that linear and parametric models are preferred in terms of universality compared to nonlinear and nonparametric models. Another important part of model building is the importance or significance of the variables. These coefficients are calculated based on the trained model and indicate the significance of each variable for the model and the task. The variables corresponding to coefficients with higher variable importance are considered to be more important to the model, and such interpretation should be combined with the spectral model of the data at hand. These values may be further used for feature selection, resulting in a more concise model. However, it should be recognized that variables with excessively high model coefficients or excessive noise should be removed from the modeling, as such variables are likely to be unreliable.
(III) Evaluation of single prediction models: models typically performs worse in prediction for unknown samples than in prediction for training/validation data, which is known as model shrinkage. In extreme cases, the statistical model can perfectly perform prediction for training/validation data, but cannot perform prediction for unknown samples at all due to overfitting. In other words, the model fits the training data too perfectly and loses universality. Therefore, it is crucial to examine predictions for unknown samples and control the error rate to ensure that the statistical model is usable in practice; this is known as model evaluation. Here, the models built in the previous steps are used to perform prediction for the test data generated by statistical sampling. If the prediction error is too large for a given predefined threshold, the statistical model should be re-modeled by modification. Regression models are evaluated by calculating a deviation between predicted and actual values; and classification and clustering models are judged based on a confusion matrix of predicted and actual values as the criterion. The confusion matrix can be used to calculate various features, including accuracy, sensitivity, and specificity.
(IV) Combination of single prediction models: the prediction values of the evaluated single prediction models are used as new input data to be substituted into the formula of the reciprocal variance. Through calculation, weight coefficients of respective single prediction models in a new ensemble model are assigned in the ensemble model, so as to build the ensemble model. In accordance with the modeling principle of single prediction models, an excessively large weight of a certain single prediction model is prevented from affecting the final prediction result, and the weight assignment is still mainly based on simple algorithms.
(V) Evaluation of the ensemble model: the ensemble model is evaluated using the same method as in (III).
(VI) Model storage: after a qualified ensemble model is built, the ensemble model is stored, along with the data preprocessing.

As shown in FIG. 28, checkpoints are set up after steps of (II) building of single prediction models, (III) evaluation of single prediction models, and (V) evaluation of the ensemble model, so as to check whether the previous step is appropriate. Specifically, at checkpoint 1, it is determined whether the model is suitable based on the validation results; and if the model is not suitable, it is required to add or replace it with a model from another modeling method. At checkpoint 2, it is checked whether the applicability of the model is suitable; and if the model's applicability is not suitable, it is required to add or replace it with a model from another modeling method for remodeling. At checkpoint 3, it is checked whether the prediction results of the ensemble model are appropriate; and if the prediction results are not appropriate, the weights of respective single models need to be recalculated and reassigned.

### (3) Model transfer

As mentioned above, it's desired to acquire the ensemble model that has good versatility, meaning that it can be used to make accurate predictions with the same ensemble model for different batches, substrates, processes, scales, and cases with spectral variation differences. In the present disclosure, such a property is sometimes referred to as model transferability.

If the model has good transferability, then in the case that all procedures of the data learning are executed correctly, the model that has been accurately tuned can also perform good prediction for new data well in the future. However, for various reasons, this is not always the case in reality. A model may be unable to perform prediction for new data, or the model's parameters may need to be further adjusted.

This phenomenon is particularly severe when Raman spectroscopy data is used. This is because the measurement performed by Raman spectroscopy is highly sensitive; minute changes in the instrument, measurement conditions, or sample preparation may be reflected as substantial shifts in the Raman shift or changes in Raman intensity. These spectral changes are unavoidable in practice, and prevent existing models from performing well in prediction for new data from new biological reactions. Therefore, the existing models may not have good model transferability between different bioreactors, or even just different batches of cell culture.

Of course, ideally, a new model may be built from scratch for each batch of cell culture processes, whenever possible. However, such operation obviously requires obtaining a large amount of training data again and re-executing the ensemble model bulding process, which is uneconomical in all aspects.

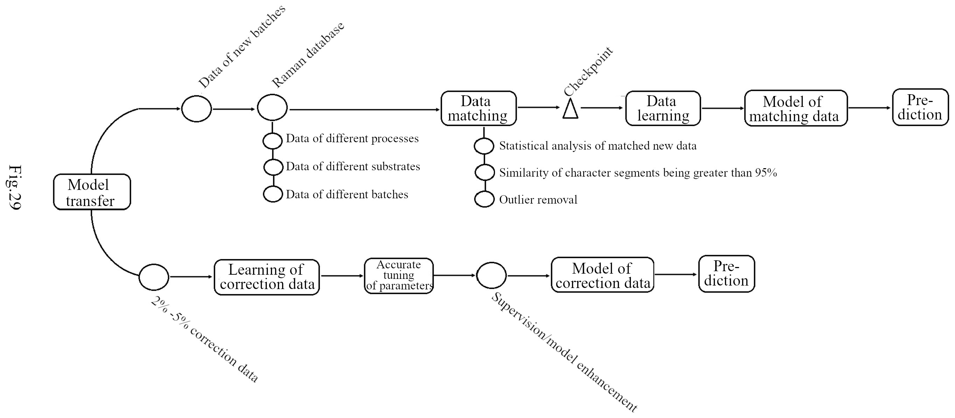

In this context, the present disclosure provides two model transfer methods. FIG. 29 illustrates two methods for model transfer, with the upper half showing the scaling matching method and the lower half showing the scaling correction method, including checking at checkpoints whether erroneous spectra are introduced during data matching and standardization. Those skilled in the art should understand that although the two methods of model transfer methods are referred to here as scale correction and scale matching, the cases requiring the model transfer are not limited to changes in the scale of biological reactions. Model transfer in cases that substrates, processes, etc., change can also be performed using the scale correction or scale matching methods.

In the present disclosure, the term "model transfer" means tuning the ensemble model built according to the method of building a machine learning ensemble model of the present disclosure to make it suitable for new batches of bioreactions. The term "new batches of bioreactions" means different batches of bioreactions, including different running batches of the same bioreaction, as well as different bioreactions (e.g., bioreactions with different substrates, processes, and/or scales), and data from new batches of bioreactions are referred to as "new-batch datasets". The new-batch dataset is different from the original training dataset originally used to build the machine learning ensemble model. The training dataset used to build the machine learning ensemble model is combined with the new-batch datasets, and the machine learning ensemble model trained in this way is called the "transferred model".

Specifically, there are two methods for performing the model transfer as follows.

Model transfer method 1: scale correction. A specified proportion of a new-batch training dataset (including spectral data and corresponding offline measurement data) is selected and added into a training set of an existing model to retrain the model, allowing the existing model to overcome differences between different batches, substrates, processes, scales, and spectral variations and acquire accurate prediction results. The specified proportion is a value selected from 1% to 10%, or a value selected from 1.5% to 7.5%, or a value selected from 2% to 5%.

Model transfer method 2: scale matching. A new batch of test data (new batch, substrate, process, scale) is collected according to the described test method, and new-batch training data, with a numerical difference from original training data collected at an identical time point as the new-batch training data in the original training dataset being less than a specified threshold, from the new-batch training dataset is incorporated into the original training dataset. Specifically, the spectral data in the new-batch training dataset is compared with the spectral data in the original training set collected at the same time, thereby obtaining the spectral data in the new-batch training dataset with the spectral numerical difference less than the specified threshold. The spectral data and corresponding offline measurement data in the new-batch training dataset are incorporated into the original training set to retrain the model, and the resulted new model is then applied to a completely new experimental environment. The specified threshold is a value selected from 1% to 10%, or a value selected from 3% to 8%, or a value selected from 4% to 6%, or 5%.

In model transfer, the retraining of the model means incorporating the new-batch training set into the original training set to form a new training set, so that the dataset of the component concentration of the cell culture solution in the bioreactor includes the new training set, the original validation dataset, and the original test set. Multiple single prediction models are built using multiple machine learning algorithms, respectively. After building a prediction model using the new training dataset, predictions are performed on the original validation dataset. The prediction results are compared with the original test dataset to obtain a square sum of prediction errors of the multiple single prediction models. Weights of the multiple single prediction models in an ensemble model are determined based on a magnitude of the square sum of prediction errors. The multiple single prediction models are combined using a weight assignment method to obtain the machine learning ensemble model.

The retraining of the model may be automated using a pre-established flow or procedure, and employing automatic calibration, one-time calibration, or timed calibration.

The following specific embodiments further illustrate the method using the machine learning ensemble model.

### Embodiment 5

First, offline measurement data and Raman spectral data are obtained through cell culture processes of wet experiments.

A CHO-K1 cell line that produces monoclonal antibodies is used in the part of the cell culture experiment. The culture conditions for this cell line in the shaker (Kuhner) for the seed phase are as follows: 36.5°C, a rotational speed of 110 rpm, a CO₂ concentration level of 6%, and Cytiva's Hyclone Actipro as a culture medium. The culture conditions for this cell line in the production culture phase are as follows: 3L and 200L reactors with initial culture volumes of 1.5L and 140L respectively, a culture temperature of 36.5°C, a pH set value of 6.90±0.25, a dissolved oxygen saturation of 40%, an initial inoculation density of 1.0×10⁶ cells/mL, and also Cytiva's Hyclone Cell Boost 7a/7b (10%/1%) as a supplemental culture medium.

Obtaining offline measurement data: in the production culture phase, a probe of a Raman device is placed into the culture solution. Samples are taken five times daily, and the viable cell density is measured using Beckman's Vi-Cell XR; glucose concentration, lactate concentration, and target protein concentration are measured using Roche's Cedex Bio analyzer; and amino acid concentrations are measured using Agilent's HPLC.

Obtaining Raman spectral data: a Raman Rxn2 analyzer equipped with an immersed optical probe (Kaiser Optical Systems) is used. The probe is installed in the 3L bioreactor (Applikon) and directly immersed in the cell culture suspension. Raman spectra of different bioreactors are recorded throughout the experiment process. For a single recorded spectrum, 30 subsequent spectra are captured with 10-second exposure time and averaged, resulting in an acquisition interval of approximately 5 minutes between acquisition operations on the bioreactor. The laser excitation wavelength is 785 nm, providing spectral coverage (Raman shift) of 100-3425 cm⁻¹. After reading the spectral data, the spectral files are converted into spectral values. The spectral file and the offline target value contained in each sample are matched one-to-one according to the time change to obtain the feature data for modeling (spectral values corresponding to different Raman shifts) and target values, and then data preprocessing begins.

After obtaining the data, the following methods are used for data preprocessing.

In this embodiment, abnormal data points are initially screened out using simple statistical methods.

S-G filtering is performed for smoothing, and data smoothing is performed by using polynomial. Based on the least squares method, "spectral burrs" are removed and random noise is eliminated.

Spike removal is performed using S-G smoothing, where a width of a smoothing window is set as follows. $n = 2 m + 1$

The n = 2m + 1 pieces of isocenter data is fitted in the window to scale the K-order polynomial and perform S-G smoothing. $\begin{matrix}{x}_{j}^{i} = {a}_{0} + {a}_{1} j + {a}_{2} {j}^{2} + ⋯ + {a}_{k} {j}^{k} \\ - m < j < m ; i \leq n\end{matrix}$

Then, Fourier transform is used for Raman shift correction and light intensity correction.

The baseline calibration may be performed using second-order differentiation, polynomial interpolation, or first-order differentiation. In this embodiment, the second-order differentiation is used for baseline calibration. ${x}_{i} , {2}_{nd} = \frac{{x}_{i + g} - 2 {x}_{i} + {x}_{i - g}}{{g}^{2}}$

Then, smoothing is performed using the first-order convolution formula of the S-G smoothing. $\frac{d \left({x}_{j}^{i + j}\right)}{d \left(j\right)} = 2 {a}_{2} j + ⋯ + {ka}_{k} {j}^{k - 1}$

In this embodiment, spectral differentiation is used as the differentiation operation to eliminate baseline drift, smooth background noise, and improve resolution.

Finally, normalization is performed using the standard normal distribution. $f \left(x\right) = \frac{1}{\sqrt{2 π}} {e}^{- \frac{{x}^{2}}{2}}$

As mentioned above, in the present disclosure, for the preprocessed data, five algorithms, i.e., Partial Least Squares (PLS), Cubist, Random Forest (RF), Support Vector Machine (SVM), and Time Series, are used to build single prediction models respectively. Then, the prediction results are processed by the Reciprocal Variance (RV) method to determine the differences between the prediction results and the validation set, thereby determining the weight of each algorithm in the ensemble model, and the new prediction result is calculated to build the machine learning ensemble model.

The same data is used to build the single prediction models. The training dataset comes from 3 fed-batch bioreactors with the same conditions, and the prediction dataset comes from a 4^{th} bioreactor with the same culture conditions. The training dataset contains 160 pieces of spectral data and corresponding offline measurement data, the validation dataset contains 50 pieces of spectral data and corresponding offline measurement data, and the test dataset contains 70 pieces of spectral data and corresponding offline measurement data.

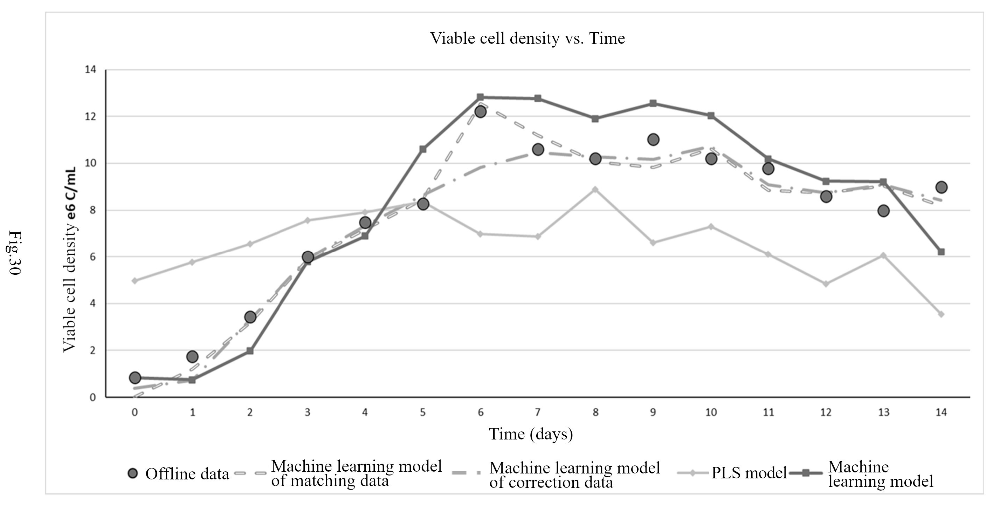

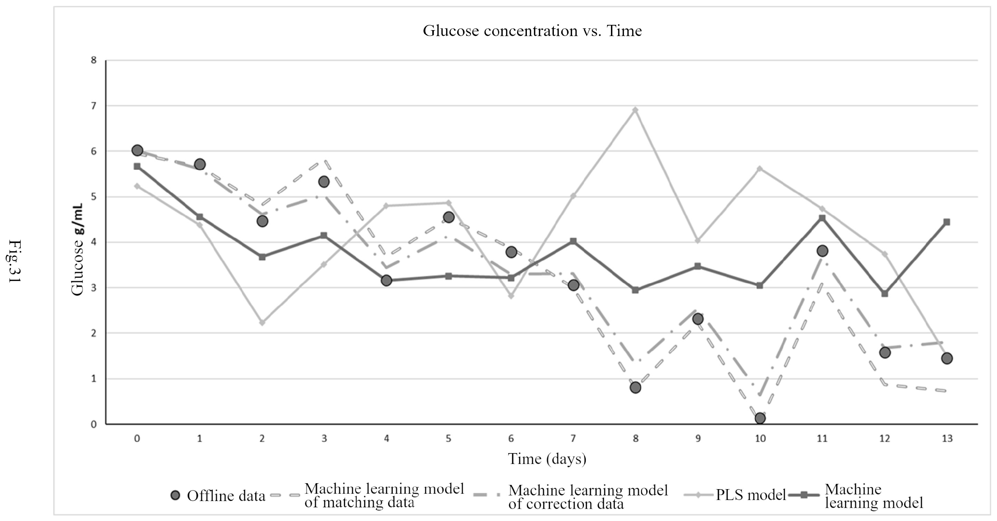

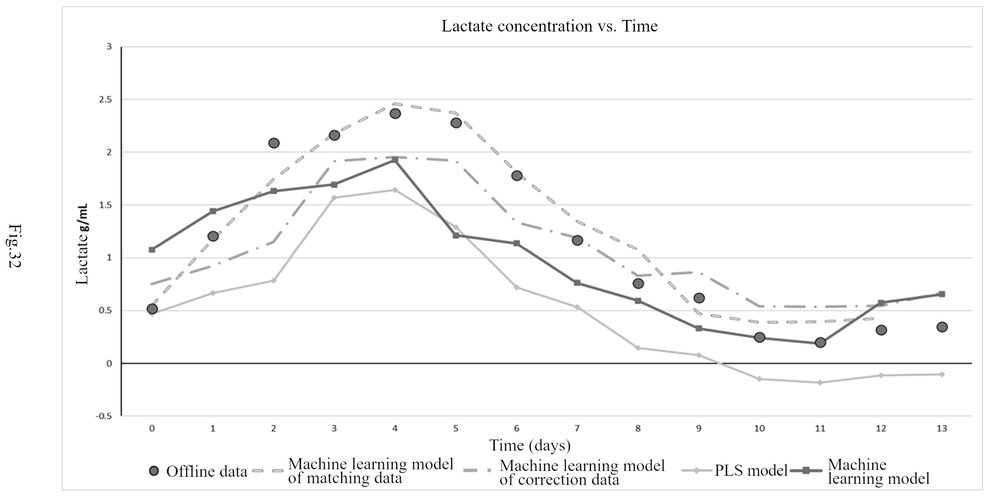

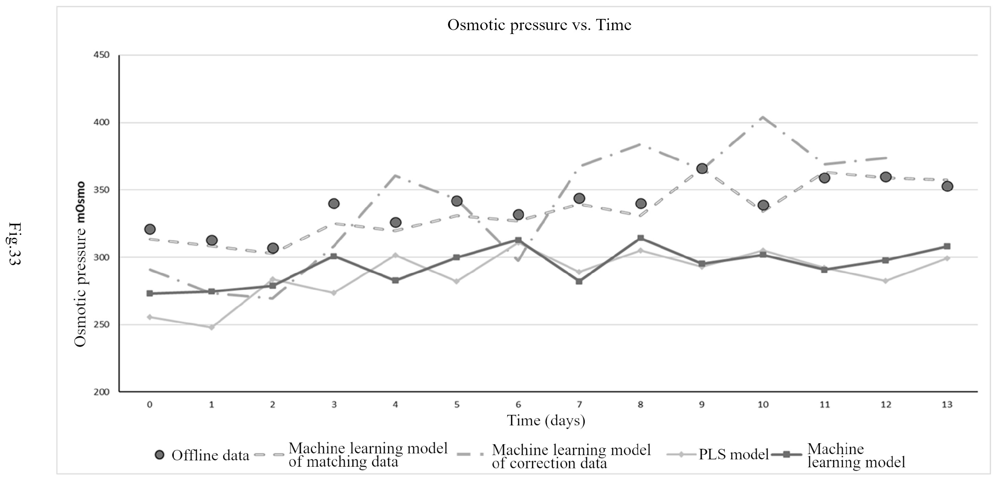

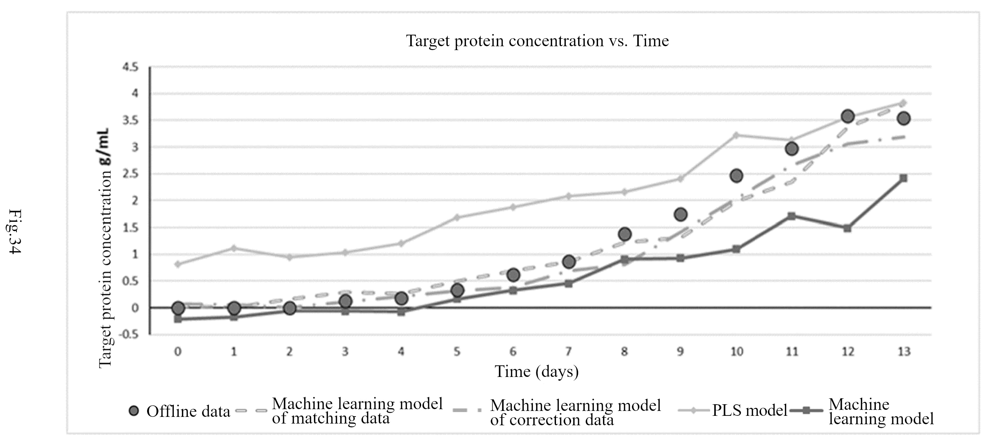

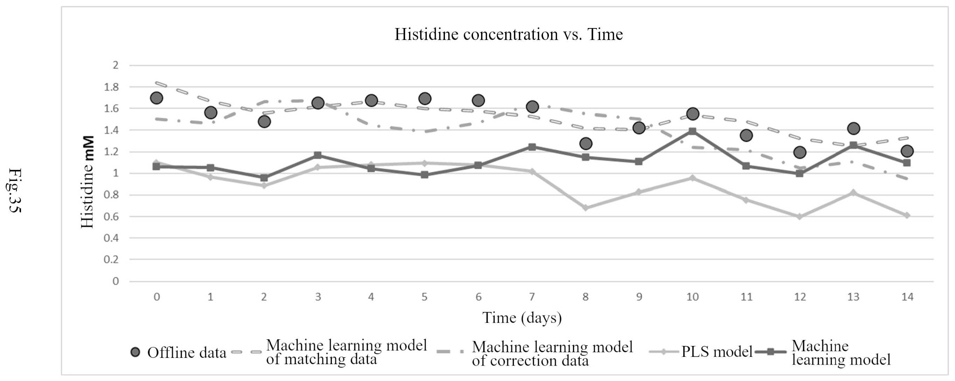

After performing predictions using the ensemble model of five algorithms, i.e., Partial Least Squares (PLS), Cubist, Random Forest (RF), Support Vector Machine (SVM), and Time Series, according to the present disclosure, the prediction results are compared with the validation set. Specific prediction results and comparisons are shown in FIGS. 30 to 35 and Table 3. In Tables 3 and 4, RMSEP means the root mean square error of predictions.

**Table 3. RMSEP data of amino acid**

| **Amino acid** | **Root mean square error of predictions (RMSEP) (mM)** |
|---|---|
| Alanine (Ala) | 0.73 |
| Arginine (Arg) | 0.27 |
| Asparagine (Asn) | 0.62 |
| Aspartate (Asp) | 0.39 |
| Cysteine (Cys) | 0.18 |
| Glutamine (Gln) | 0.08 |
| glutamate (Glu) | 1.07 |
| Glycine (Gly) | 0.60 |
| Histidine (His) | 0.11 |
| Isoleucine (Ile) | 0.14 |
| Leucine (Leu) | 0.20 |
| Lysine (Lys) | 0.50 |
| Methionine (Met) | 0.18 |
| Phenylalanine (Phe) | 0.34 |
| Proline (Pro) | 0.20 |
| Serine (Ser) | 0.85 |
| Threonine (Thr) | 0.82 |
| Tryptophan (Trp) | 0.10 |
| Tyrosine (Tyr) | 0.38 |
| Valine (Val) | 0. 79 |

To acquire quantitative comparison results, Table 4 shows the results of comparing the PLS model with the machine learning ensemble model of the present disclosure using the Leave-One-Out cross verification method.

**Table 4**

| Process parameters | Range | RMSEP* of PLS model | RMSEP of machine learning model | RMSEP of PLS model percentage | RMSEP of machine learning model percentage |
|---|---|---|---|---|---|
| | e6 c/mL or g/L or mM or mOsmo | | | | |
| | | e6 c/mL or g/L or mM or mOsmo | | % | |
| Viable cell density | 0.8~13.0 | 3.51 | 0.85 | 50.9 | 12.3 |
| Glucose concentration | 0.5~7.0 | 1.12 | 0.32 | 29.9 | 8.5 |
| Lactate concentration | 0.1~2.5 | 0.67 | 0.22 | 51.5 | 16.9 |
| Osmotic pressure | 245~430 | 52.14 | 8.45 | 15.4 | 2.5 |
| Target protein concentration | 0~4.0 | 1.18 | 0.29 | 59.1 | 14.5 |
| Histidine concentration | 0.5~2 | 0.21 | 0.13 | 16.8 | 10.4 |

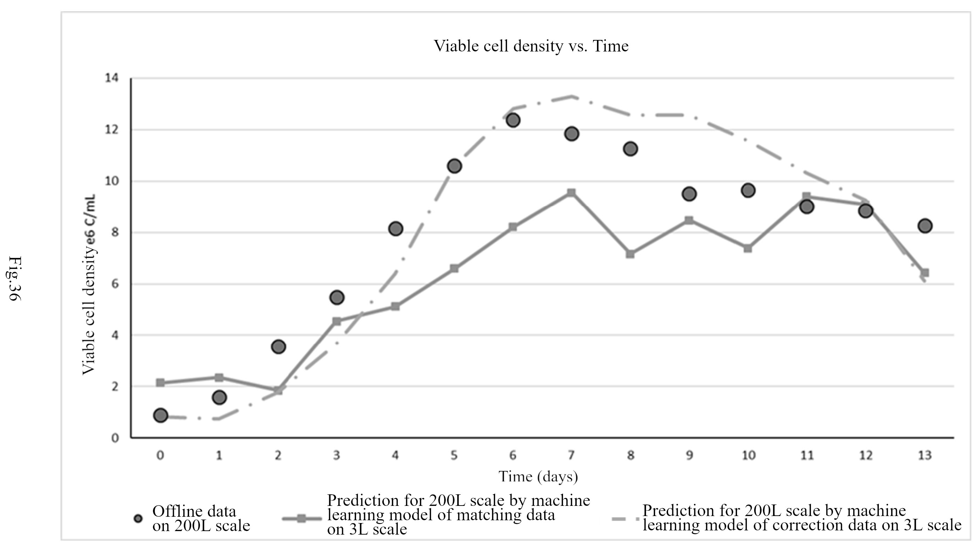

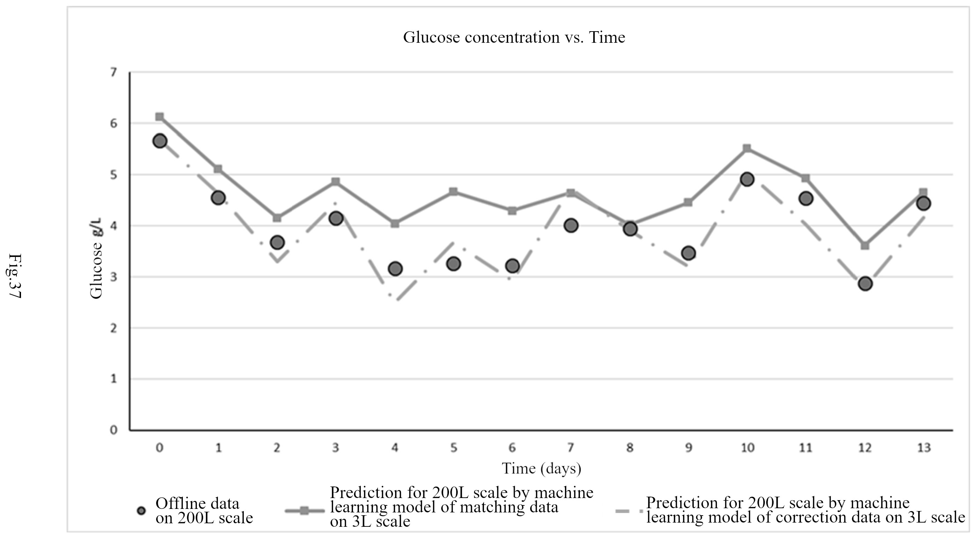

The model transferability of the method of the present disclosure is further verified as follows. FIGS. 36 and 37 are diagrams showing prediction effects for two indicators (viable cell density and glucose concentration) obtained by prediction after the model is transferred between different scales.

The two transfer methods are compared in Table 5, and it is found that the model transfer method of scale correction is more suitable for practical applications.

**Table 5**

| Process parameters | Range e6 c/mL or g/L or mM or mOsmo | RMSEP of machine learning model of matched data e6 c/mL or g/L or mM or | RMSEP of machine learning model of corrected data | RMSEP of machine learning model of matched data percentage % | RMSEP of machine learning model of corrected data percentage |
|---|---|---|---|---|---|
| Viable cell density | 0.8~13.0 | 0.63 | 0.79 | 9.1 | 11.4 |
| Glucose concentration | 0.5~7.0 | 0.18 | 0.24 | 4.8 | 6.4 |
| Lactate concentration | 0.1~2.5 | 0.12 | 0.18 | 9.2 | 13.8 |
| Osmotic pressure | 245~430 | 5.32 | 7.32 | 1.5 | 2.1 |
| Titer of target protein | 0~4.0 | 0.14 | 0.17 | 7 | 8.5 |
| Histidine concentration | 0.5~2 | 0.09 | 0.11 | 7.2 | 8.8 |

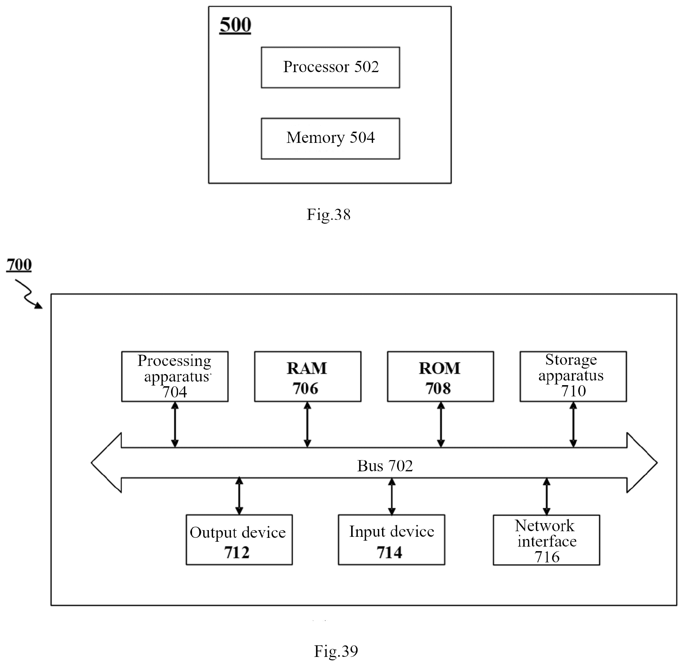

The present disclosure further provides a computer device including a memory and a processor, where the memory stores thereon a computer program that can run on the processor, and the computer program, when executed by the processor, implements the steps of the automatic material supplementing method based on the real-time dynamic measurement result according to any embodiment of the present disclosure. Specifically, referring to FIG. 38, a schematic block diagram of a computer device 500 according to some embodiments of the present disclosure is shown. As shown in FIG. 38, the computer device 500 includes the processor 502 and the memory 504. The processor 502 may be, for example, a central processing unit (CPU) of the computer device 500. The processor 502 may be any type of general-purpose processor, or may be a processor specifically designed for automatic material supplementing, such as an application-specific integrated circuit ("ASIC"). The memory 504 may be coupled to the processor 502 and may include various computer-readable media accessible by the processor 502. In various embodiments, the memory 504 described herein may include volatile and non-volatile media, removable and non-removable media. For example, the memory 504 may include any combination of the following: random access memory ("RAM"), dynamic RAM ("DRAM"), static RAM ("SRAM"), read-only memory ("ROM"), flash memory, cache memory and/or any other type of non- transitory computer-readable medium. The memory 504 may store thereon a computer program that can run on the processor 502, and the computer program, when executed by the processor 502, implements the automatic material supplementing method based on the real-time dynamic measurement result (e.g., the methods 100, 100') according to any embodiment of the present disclosure.

The present disclosure further provides a non-transitory computer-readable storage medium storing a computer program thereon, and the computer program, when executed by a processor, implements the steps of the automatic material supplementing method based on the real-time dynamic measurement result according to any embodiment of the present disclosure.

The present disclosure further provides a computer program product that may include instructions that, when executed by a processor, can implement the automatic material supplementing method based on the real-time dynamic measurement result according to any embodiment of the present disclosure. The instructions may be any instruction set that will be executed directly by the processor, such as machine code, or any instruction set that will be executed indirectly by the processor, such as scripts. The instruction may be stored in object code format for direct processing by the processor, or stored in any other computer language, including scripts or collections of standalone source code modules that are interpreted on demand or compiled in advance.

FIG. 39 shows a schematic block diagram of a computer system 700 on which the embodiments of the present disclosure may be implemented. The computer system 700 includes a bus 702 or other communication mechanism for communicating information, and a processing apparatus 704 coupled to the bus 702 for processing information. The computer system 700 further includes a memory 706 coupled to the bus 702 for storing instructions to be executed by the processing apparatus 704. The memory 706 may be a random access memory (RAM) or other dynamic storage device. The memory 706 may also be used to store temporary variables or other intermediate information during execution of instructions to be executed by the processing apparatus 704. The computer system 700 further includes a read-only memory (ROM) 708 or other static storage device coupled to the bus 702 for storing static information and instructions for the processing apparatus 704. A storage apparatus 710, such as a magnetic disk or an optical disk, is provided and coupled to the bus 702 for storing information and instructions. The computer system 700 may be coupled via the bus 702 to an output device 712 for providing output to a user, such as, but not limited to, a display (such as a cathode ray tube (CRT) or liquid crystal display (LCD)), a speaker, etc. An input device 714, such as a keyboard, a mouse, and a microphone, is coupled to the bus 702 for transmitting information and command selections to the processing apparatus 704. The computer system 700 can perform the embodiments of the present disclosure. Consistent with certain implementations of the present disclosure, the computer system 700 provides results in response to the processing apparatus 704 executing one or more sequences of one or more instructions contained in the memory 706. Such instructions may be read from another computer-readable medium, such as the storage apparatus 710, into the memory 706. The execution of the instruction sequence contained in the memory 706 causes the processing apparatus 704 to perform the method described herein. Alternatively, the present teaching may be implemented using hardwired circuitry instead of or in combination with software instructions. Therefore, the implementation of the present disclosure is not limited to any particular combination of hardware circuitry and software. In various embodiments, the computer system 700 may be connected across a network to one or more other computer systems like the computer system 700 via a network interface 716 to form a networked system. This network may include a private network or a public network such as the Internet. In the networked system, one or more computer systems may store data and supply data to other computer systems. As used herein, the term "computer-readable medium" means any medium that participates in providing instructions to the processing apparatus 704 for execution. The medium may take many forms, including but not limited to non-volatile media, volatile media, and transmission media. The non-volatile media include, for example, optical disks or magnetic disks such as the storage apparatus 710. The volatile media include dynamic memory such as the memory 706. The transmission media include coaxial cable, copper wire and optical fiber, including wiring comprising the bus 702. Common forms of computer-readable media or computer program products include, for example, floppy disks, flexible disks, hard disks, magnetic tapes, or any other magnetic media, CD-ROMs, digital video discs (DVDs), Blu-ray discs, any other optical media, thumb drives, memory cards, RAM, PROMs and EPROMs, fast EPROMs, any other memory chips or cartridges, or any other tangible media from which a computer can read. Various forms of computer-readable media may be involved when carrying one or more sequences of one or more instructions to the processing apparatus 704 for execution. For example, instructions may initially be carried on a magnetic disk of a remote computer, and the remote computer may load the instructions into its dynamic memory and send the instructions over a telephone line using a modem. The computer system 700's local modem may receive data over the telephone line and convert the data into infrared signals using an infrared transmitter. A infrared detector coupled to the bus 702 may receive the data carried in the infrared signals and place the data on the bus 702. The bus 702 carries the data to the memory 706, and the processing apparatus 704 retrieves the instructions from the memory 706 and executes the instructions. Optionally, the instructions received by the memory 706 may be stored on the storage apparatus 710 before or after execution by the processing apparatus 704.

According to various embodiments, instructions configured to be executed by the processing apparatus to perform the method are stored on computer-readable media. The computer-readable media may be devices that store digital information. For example, the computer-readable media include compact disc read-only memory (CD-ROM) as known in the art for storing software. The computer-readable media are accessed by a processor adapted to execute instructions configured to be executed.

The foregoing has described one or more exemplary embodiments of the present disclosure. Other embodiments are within the scope of the appended claims. In some cases, the actions or steps described in the claims may be performed in an order different from that in the embodiments and still achieve the desired results. Furthermore, the processes depicted in the accompanying drawings do not necessarily require a specific or sequential order to achieve the desired results. In some implementations, multitasking and parallel processing are also possible or may be advantageous.

The systems, apparatuses, modules, or units described in the above embodiments may be implemented by computer chips or entities, or by products with certain functions. A typical implementation device is a server system. Of course, the present disclosure does not exclude the possibility that, with the future development of computer technologies, the computer that implements the functions of the above embodiments may be, for example, a personal computer, a laptop computer, an in-vehicle human-computer interaction device, a cellular phone, a camera phone, a smartphone, a personal digital assistant, a media player, a navigation device, an email device, a game console, a tablet computer, a wearable device, or any combination of these devices.

While one or more embodiments of the present disclosure provide the steps of operation for the methods described in the embodiments or flowcharts, more or fewer steps may be included based on conventional or non-inventive means. The order of steps listed in the embodiments is only one manner in many execution orders of steps, and does not represent the only execution order. When executed in a real apparatus or terminal product, sequential or parallel execution may be performed according to the method in the embodiments or shown in the accompanying drawings (e.g., in a parallel processor or multi-threaded processing environment, or even a distributed data processing environment).

The terms "comprising," "including," or any other variations thereof are intended to cover non-exclusive inclusion, such that a process, method, product, or device that includes a list of elements includes not only those elements but also other elements not expressly listed, or elements inherent to such process, method, product, or device. Without further restrictions, it is not excluded that other identical or equivalent elements may be present in the process, method, product or device that includes the aforementioned elements. For example, the words such as "first" and "second" are used to indicate names, but do not indicate any specific order.

For ease of description, the above apparatuses are described in the manner of being divided into various modules in terms of function. Of course, when implementing one or more embodiments of the present disclosure, the functions of respective modules may be implemented in one or more software and/or hardware, or the module that implements the same function may be implemented by a combination of multiple sub-modules or sub-units, etc. The apparatus embodiments described above are merely illustrative. For example, the division of the units is only a logical functional division, and there may be other division manners in actual implementation. For example, multiple units or components may be combined or integrated into another system, or some features may be ignored or not executed. Another point is that the mutual coupling or direct coupling or communication connection shown or discussed may be an indirect coupling or communication connection through some interface, apparatus or unit, and may be electrical, mechanical or in other forms.

The present disclosure is described with reference to flowcharts and/or block diagrams of methods, apparatus (systems), and computer program products according to embodiments of the present disclosure. It should be understood that each flow and/or block in the flowcharts and/or block diagrams, as well as combinations of flows and/or blocks in the flowchart and/or block diagrams, may be implemented by computer program instructions. These computer program instructions may be provided to a processor of a general-purpose computer, a special-purpose computer, an embedded processor, or other programmable data processing device to produce a machine, such that the instructions, which are executed by the processor of the computer or other programmable data processing device, produce an apparatus for implementing the functions specified in one or more flows of the flowcharts and/or one or more blocks of the block diagrams.

These computer program instructions may also be stored in a computer-readable storage that can direct a computer or other programmable data processing device to operate in a particular manner, such that the instructions stored in the computer-readable storage produce an article of manufacture including an instruction apparatus that implements the functions specified in one or more flows of the flowcharts and/or one or more blocks of the block diagrams. These computer program instructions may also be loaded onto a computer or other programmable data processing device to cause a series of operational steps to be performed on the computer or other programmable device to produce a computer-implemented process, such that the instructions, which are executed on the computer or other programmable device, provide steps for implementing the functions specified in one or more flows of the flowcharts and/or one or more blocks of the block diagrams.

Those skilled in the art will understand that one or more embodiments of the present disclosure may take the form of a completely hardware embodiment, a completely software embodiment, or an embodiment combining software and hardware aspects. Furthermore, one or more embodiments of the present disclosure may take the form of a computer program product implemented on one or more computer-usable storage media (including, but not limited to, disk storage, CD-ROM, optical storage, etc.) containing computer-usable program code.

One or more embodiments of the present disclosure may be described in the general context of computer-executable instructions that are executed by a computer, such as program modules. Generally, the program modules include routines, programs, objects, components, data structures, etc., that perform specific tasks or implement specific abstract data types. One or more embodiments of the present disclosure may also be practiced in distributed computing environments in which tasks are performed by remote processing devices connected over a communication network. In the distributed computing environments, the program modules may reside on local and remote computer storage media, including storage devices.

The same or similar parts between the various embodiments of the present disclosure may be referred to mutually, and each embodiment focuses on describing the differences from other embodiments. In particular, the apparatus embodiments are basically similar to the method embodiments, so the description thereof is relatively simple. For relevant details, the description of the method embodiments may be referred to. In the descriptions of the present disclosure, references to terms such as "one embodiment," "some embodiments," "an embodiment," "example," "specific example," or "some examples" mean that a specific feature, structure, material, or characteristic described in connection with that embodiment or example is included in at least one embodiment or example of the present disclosure. In the present disclosure, the illustrative expressions of the above-mentioned terms do not necessarily refer to the same embodiment or example. Furthermore, the specific features, structures, materials, or characteristics described may be combined in a suitable manner in any one or more embodiments or examples. Furthermore, those skilled in the art can combine and integrate the different embodiments or examples, as well as the features of the different embodiments or examples described in the present disclosure, without contradiction.

Furthermore, when used in the present disclosure, the terms "herein", "above", "below", "hereinafter," "hereinafter" and similar terms shall refer to the entire of the present disclosure instead of any particular part thereof. Furthermore, unless otherwise expressly stated or otherwise understood in the context in which they are used, the conditional language used herein, such as "can", "may", "for example", "such as", etc., are generally intended to express that some embodiments include, while other embodiments do not include, some features, elements, and/or states. Therefore, such conditional languages are not generally intended to imply that one or more embodiments require the features, elements, and/or states in any way, or whether such features, elements, and/or states are included, or such features, elements, and/or states are implemented in any particular embodiment.

The above description includes merely examples of one or more embodiments of the present disclosure and is not intended to limit one or more embodiments of the present disclosure. For those skilled in the art, one or more embodiments of the present disclosure may have various modifications and variations. Any modifications, equivalent substitutions, or improvements made within the spirit and principles of the present disclosure shall be included within the scope of the claims.

## Claims

1. An automatic material supplementing method based on a real-time dynamic measurement result, comprising:
collecting the real-time dynamic measurement result, and calculating a rate of change of each component within a unit time, wherein the rate of change is a consumption rate or a generation rate; selecting a component having the highest rate of change as a marker for a current time period;
collecting historical measurement data, and calculating a cell growth rate in a logarithmic growth phase and a target product expression rate in a culture harvest phase; taking, as a component concentration set value for the current time period of the logarithmic growth phase, a concentration level of the marker when the cell growth rate in the logarithmic growth phase is highest; taking, as a component concentration set value for the current time period of the culture harvest phase, a concentration level of the marker when the target product expression rate in the culture harvest phase is highest; and
calculating an addition amount of the marker for the current time period based on the rate of change of the marker, and calculating an addition amount of a culture medium based on the addition amount of the marker to obtain a material supplementing strategy.

2. The automatic material supplementing method based on the real-time dynamic measurement result of claim 1, wherein a step of selecting the marker comprises:
collecting a current real-time dynamic measurement result, and calculating a rate of change of each component within a current unit time;
collecting a historical real-time dynamic measurement result, and calculating a rate of change of each component within a historical unit time; and
combining the rate of change of each component within the current unit time with the rate of change of each component within the historical unit time, and selecting a component having the highest rate of change therefrom as the marker for the current time period.

3. The automatic material supplementing method based on the real-time dynamic measurement result of claim 1 or 2, wherein the historical measurement data is historical measurement data of a fermentation process with the same cell culture process mode as a present fermentation process.

4. The automatic material supplementing method based on the real-time dynamic measurement result of any one of claims 1 to 3, wherein a step of calculating the addition amount of the culture medium comprises: ${m}_{suppl} = \left({G}_{set}-{G}_{rt}\right) \times {V}_{cs} / {G}_{suppl} ,$ wherein mₛᵤₚₚₗ represents an amount of a supplemental culture medium to be added, Gₛₑₜ represents a set concentration of the marker, Gᵣₜ represents a current concentration of the marker measured by analyzing a Raman spectrum in real time with a model, V_{cs} represents a volume of a cell culture solution in a bioreactor, and Gₛᵤₚₚₗ represents a concentration of the marker in the supplemental culture medium.

5. The automatic material supplementing method based on the real-time dynamic measurement result of any one of claims 1 to 4, wherein the supplemental culture medium comprises a specific proportion of the marker and another component, and an addition amount of the other component is obtained based on the addition amount of the marker.

6. The automatic material supplementing method based on the real-time dynamic measurement result of any one of claims 1 to 5, further comprising writing the material supplementing strategy into a control system after obtaining the material supplementing strategy; and
optionally, the control system is configured to perform material supplementing instantaneously in a dose-wise manner by cooperating with a peristaltic pump hardware upon receiving an numerical instruction on a supplemental amount, a set value, and a time point for material supplementing.

7. An automatic material supplementing apparatus based on a real-time dynamic measurement result, comprising:
a marker selecting unit configured to collect the real-time dynamic measurement result, and to calculate a rate of change of each component within a unit time, wherein the rate of change is a consumption rate or a generation rate, and to select a component having the highest rate of change as a marker for a current time period;
a marker concentration setting unit configured to collect historical measurement data and to calculate a cell growth rate in a logarithmic growth phase and a target product expression rate in a culture harvest phase, to take, as a component concentration set value for the current time period of the logarithmic growth phase, a concentration level of the marker when the cell growth rate in the logarithmic growth phase is highest, and to take, as a component concentration set value for the current time period of the culture harvest phase, a concentration level of the marker when the target product expression rate in the culture harvest phase is highest; and
a supplemental amount calculation unit configured to calculate an addition amount of the marker for the current time period based on the rate of change of the marker, and to calculate an addition amount of a culture medium based on the addition amount of the marker to obtain a material supplementing strategy.

8. The automatic material supplementing apparatus based on the real-time dynamic measurement result of claim 7, wherein a step of selecting the marker in the marker selecting unit comprises:
collecting a current real-time dynamic measurement result, and calculating a rate of change of each component within a current unit time;
collecting a historical real-time dynamic measurement result, and calculating a rate of change of each component within a historical unit time; and
combining the rate of change of each component within the current unit time with the rate of change of each component within the historical unit time, and selecting a component having the highest rate of change therefrom as the marker for the current time period;
optionally, in the marker concentration setting unit, the historical measurement data is historical measurement data of a fermentation process with the same cell culture process mode as a present fermentation process;
optionally, in the supplemental amount calculation unit, a step of calculating the addition amount of the culture medium comprises: ${m}_{suppl} = \left({G}_{set}-{G}_{rt}\right) \times {V}_{cs} / {G}_{suppl}$
wherein mₛᵤₚₚₗ represents an amount of a supplemental culture medium to be added, Gₛₑₜ represents a set concentration of the marker, Gᵣₜ represents a current concentration of the marker measured by analyzing a Raman spectrum in real time with a model, V_{cs} represents a volume of a cell culture solution in a bioreactor, and Gₛᵤₚₚₗ represents a concentration of the marker in the supplemental culture medium;
optionally, in the supplemental amount calculation unit, the supplemental culture medium comprises a specific proportion of the marker and another component, and an addition amount of the other component is obtained based on the addition amount of the marker;
optionally, in the supplemental amount calculation unit, writing the material supplementing strategy into a control system is further comprised after obtaining the material supplementing strategy; and
optionally, the control system is configured to perform material supplementing instantaneously in a dose-wise manner by cooperating with a peristaltic pump hardware upon receiving an numerical instruction on a supplemental amount, a set value, and a time point for material supplementing.

9. An automatic material supplementing method based on a real-time dynamic measurement result, comprising:
obtaining a historical real-time dynamic measurement result, calculating a historical rate of change of each component based on the historical real-time dynamic measurement result, and selecting one component or a combination of multiple components as a marker based on the historical rate of change of each component;
collecting a current real-time dynamic measurement result, and calculating a real-time measured concentration of the marker for a current time period based on the current real-time dynamic measurement result; and
obtaining a material supplementing strategy by at least one of:
calculating a cell growth rate in a logarithmic growth phase and a target product expression rate in a culture harvest phase based on the historical real-time dynamic measurement result, taking, as a component concentration set value of the marker for the current time period, a concentration level of the marker when the cell growth rate in the logarithmic growth phase is highest in a case that the current time period falls into the logarithmic growth phase, and taking, as a component concentration set value of the marker for the current time period, a concentration level of the marker when the target product expression rate in the culture harvest phase is highest in a case that the current time period falls into the culture harvest phase, and calculating an addition amount of a supplemental culture medium for the current time period based on a difference between the real-time measured concentration of the marker for the current time period and the component concentration set value of the marker for the current time period to obtain a first material supplementing strategy; or
calculating a current rate of change of the marker based on the real-time measured concentration of the marker for the current time period, and calculating an addition amount of the supplemental culture medium for the current time period based on the current rate of change of the marker to obtain a second material supplementing strategy.

10. The automatic material supplementing method based on the real-time dynamic measurement result of claim 9, further comprising:
calculating multiple historical rates of change of each component within multiple historical time periods based on the historical real-time dynamic measurement result; and
combining the multiple historical rates of change of each component to obtain a combined historical rate of change of each component, and selecting one component or a combination of multiple components as the marker based on the combined historical rate of change of each component.

11. The automatic material supplementing method based on the real-time dynamic measurement result of claim 9 or 10, wherein the historical real-time dynamic measurement result and the current real-time dynamic measurement result are obtained from different fermentation processes with the same cell culture process mode.

12. The automatic material supplementing method based on the real-time dynamic measurement result of any one of claims 9 to 11, wherein calculating the addition amount of the supplemental culture medium for the current time period based on the difference between the real-time measured concentration of the marker for the current time period and the component concentration set value of the marker for the current time period comprises one or more of:
in a case that the rate of change of the marker is expressed as a consumption rate, ${m}_{suppl} = \left({G}_{set}-{G}_{rt}\right) \times {V}_{cs} / {G}_{suppl} ,$
wherein mₛᵤₚₚₗ represents the addition amount of the supplemental culture medium for the current time period, Gₛₑₜ represents the component concentration set value of the marker for the current time period, Gᵣₜ represents the real-time measured concentration of the marker for the current time period, V_{cs} represents a volume of a cell culture solution in a bioreactor, and Gₛᵤₚₚₗ represents a concentration of the marker in the supplemental culture medium; or
in a case that the rate of change of the marker is expressed as a generation rate, ${m}_{suppl} = k \times \left({A}_{rt}-{A}_{set}\right) \times {V}_{cs} / {B}_{suppl} ,$
wherein mₛᵤₚₚₗ represents the addition amount of the supplemental culture medium for the current time period, Aₛₑₜ represents the component concentration set value of the marker for the current time period, Aᵣₜ represents the real-time measured concentration of the marker for the current time period, V_{cs} represents the volume of the cell culture solution in the bioreactor, Bₛᵤₚₚₗ represents a concentration of a culture medium component, from which the marker is converted, in the supplemental culture medium, and k represents a conversion coefficient from the concentration of the marker to the concentration of the culture medium component.

13. The automatic material supplementing method based on the real-time dynamic measurement result of any one of claims 9 to 12, wherein calculating the addition amount of the supplemental culture medium for the current time period based on the current rate of change of the marker comprises one or more of:
in a case that the rate of change of the marker is expressed as a consumption rate, ${m}_{suppl} = R \times {V}_{cs} / {G}_{suppl} , R = {G}_{t 0} - {G}_{rt} ,$
wherein mₛᵤₚₚₗ represents the addition amount of the supplemental culture medium for the current time period, R is the current rate of change of the marker, V_{cs} represents a volume of a cell culture solution in a bioreactor, Gₛᵤₚₚₗ represents a concentration of the marker in the supplemental culture medium, Gₜ₀ represents a real-time measured concentration of the marker for a previous time period, and Gᵣₜ represents the real-time measured concentration of the marker for the current time period; or
in a case that the rate of change of the marker is expressed as a generation rate, ${m}_{suppl} = k \times R \times {V}_{cs} / {B}_{suppl} , R = {A}_{rt} - {A}_{t 0} ,$
wherein mₛᵤₚₚₗ represents the addition amount of the supplemental culture medium for the current time period, R is the current rate of change of the marker, V_{cs} represents the volume of the cell culture solution in the bioreactor, Aₜ₀ represents the real-time measured concentration of the marker for the previous time period, Aᵣₜ represents the real-time measured concentration of the marker for the current time period, Bₛᵤₚₚₗ represents a concentration of a culture medium component, from which the marker is converted, in the supplemental culture medium, and k represents a conversion coefficient from the concentration of the marker to the concentration of the culture medium component.

14. The automatic material supplementing method based on the real-time dynamic measurement result of any one of claims 9 to 13, comprising:
determining a material supplementing strategy to be applied based on the first material supplementing strategy and the second material supplementing strategy,
optionally, the material supplementing strategy to be applied is determined to be an average of the first material supplementing strategy and the second material supplementing strategy,
optionally, the material supplementing strategy to be applied is selected from the first material supplementing strategy and the second material supplementing strategy based on a phase into which the current time period falls, and
optionally, in a case that the current time period falls into a target product rapid production phase, the first material supplementing strategy is selected as the material supplementing strategy to be applied; and in a case that the current time period falls into the logarithmic growth phase, the second material supplementing strategy is selected as the material supplementing strategy to be applied.

15. The automatic material supplementing method based on the real-time dynamic measurement result of any one of claims 9 to 14, wherein calculating the addition amount of the supplemental culture medium for the current time period comprises:
calculating an addition amount of the marker or of a culture medium component, from which the marker is converted, for the current time period, based on the difference between the real-time measured concentration of the marker for the current time period and the component concentration set value of the marker for the current time period, or based on the current rate of change of the mark; and
calculating the addition amount of the supplemental culture medium for the current time period based on the addition amount of the marker or of the culture medium component, from which the marker is converted, for the current time period.

16. The automatic material supplementing method based on the real-time dynamic measurement result of any one of claims 9 to 15, wherein the supplemental culture medium comprises a specific proportion of the marker and another component, and an addition amount of the other component is obtained based on an addition amount of the marker,
optionally, the automatic material supplementing method comprises writing the material supplementing strategy into a control system, wherein the control system is configured to perform material supplementing instantaneously in a dose-wise manner by cooperating with a peristaltic pump hardware upon receiving an numerical instruction on the material supplementing strategy,
optionally, a real-time measured concentration of a reference component for the current time period is compared with a set value, and the automatic material supplementing method is not performed responsive to the real-time measured concentration of the reference component for the current time period being not lower than the set value, or the automatic material supplementing method is performed responsive to the real-time measured concentration of the reference component for the current time period being lower than the set value.

17. The automatic material supplementing method based on the real-time dynamic measurement result of any one of claims 9 to 16, wherein the current real-time dynamic measurement result is online spectral data, and calculating the real-time measured concentration of the marker for the current time period based on the current real-time dynamic measurement result comprises inputting the current real-time dynamic measurement result into a trained text convolutional model to obtain the real-time measured concentration of the marker for the current time period,
wherein the text convolution model is trained by taking the online spectral data of an indicator of a cell culture solution in a bioreactor at multiple different time points as samples and taking offline target values of the indicator measured by sampling the cell culture solution as labels, and the text convolution model is configured to receive real-time measured spectral data of the indicator of a cell culture solution to be predicted and output a prediction value of the indicator.

18. The automatic material supplementing method based on the real-time dynamic measurement result of claim 17, wherein the text convolution model is configured to:
set a word vector mapping layer and perform feature dimensionality reduction; and
obtain, using a fully connected structure, the prediction value of the indicator after averaging over a spatial dimension.

19. The automatic material supplementing method based on the real-time dynamic measurement result of claim 18, wherein the text convolution model is configured to: further perform neighbor feature sampling after setting the word vector mapping layer and performing the feature dimensionality reduction.

20. The automatic material supplementing method based on the real-time dynamic measurement result of any one of claims 9 to 16, wherein calculating the real-time measured concentration of the marker for the current time period based on the current real-time dynamic measurement result comprises inputting the current real-time dynamic measurement result into a machine learning ensemble model for predicting a component concentration in a cell culture solution to obtain the real-time measured concentration of the marker for the current time period,
wherein the machine learning ensemble model is built by:
obtaining a dataset of a component concentration of a cell culture solution in a bioreactor, the dataset comprising a training dataset, a validation dataset, and a test dataset;
building multiple single prediction models by using multiple machine learning algorithms, respectively, wherein each of the multiple single prediction models performs prediction on the test dataset after being trained with the training dataset and validated with the validation dataset;
obtaining a square sum of prediction errors of the multiple single prediction models by comparing prediction results of the multiple single prediction models with the test dataset, and determining weights of the multiple single prediction models in the machine learning ensemble model based on a magnitude of the square sum of the prediction errors; and
obtaining the machine learning ensemble model by combining the multiple single prediction models through a weight assignment method.

21. The automatic material supplementing method based on the real-time dynamic measurement result of any one of claims 9 to 16, wherein calculating the real-time measured concentration of the marker for the current time period based on the current real-time dynamic measurement result comprises inputting the current real-time dynamic measurement result into a machine learning ensemble model for predicting a component concentration in a cell culture solution to obtain the real-time measured concentration of the marker for the current time period,
wherein the machine learning ensemble model is a transferred model obtained by performing model transfer relative to an original model,
wherein the original model is built by:
obtaining a dataset of a component concentration of a cell culture solution in a bioreactor, the dataset comprising a training dataset, a validation dataset, and a test dataset;
building multiple single prediction models by using multiple machine learning algorithms, respectively, wherein each of the multiple single prediction models performs prediction on the test dataset after being trained with the training dataset and validated with the validation dataset;
obtaining a square sum of prediction errors of the multiple single prediction models by comparing prediction results of the multiple single prediction models with the test dataset, and determining weights of the multiple single prediction models in the original model based on a magnitude of the square sum of the prediction errors; and
obtaining the original model by combining the multiple single prediction models through a weight assignment method;
wherein the model transfer comprises:
obtaining an original dataset used for building the original model, and a new-batch training dataset of the component concentration of a cell culture solution of a new batch of biological reactions, the original dataset comprising an original training dataset, an original validation dataset, and an original test dataset,
obtaining a new training dataset by performing scale correction or scale matching on the new-batch training dataset and the original training dataset,
building multiple single prediction models by using multiple machine learning algorithms, respectively, with the new training dataset, the original validation dataset, and the original test dataset, wherein each of the multiple single prediction models performs prediction on the original test dataset after being trained with the new training dataset and validated with the original validation dataset,
obtaining a square sum of prediction errors of the multiple single prediction models by comparing prediction results of the multiple single prediction models with the original test dataset, and determining weights of the multiple single prediction models in the transferred model based on a magnitude of the square sum of the prediction errors; and
obtaining the transferred model by combining the multiple single prediction models through a weight assignment method,
wherein the scale correction comprises incorporating a specified proportion of data from the new-batch training dataset into the original training dataset used for building the original model; and
the scale matching comprises incorporating new-batch training data, with a numerical difference from original training data collected at an identical time point as the new-batch training data in the original training dataset being less than a specified threshold, from the new-batch training dataset into the original training dataset.

22. The automatic material supplementing method based on the real-time dynamic measurement result of any one of claims 20 to 21, wherein the dataset of the component concentration comprises online Raman spectral data and corresponding offline measurement data thereof, and a sampling time point of the offline measurement data is matched with corresponding online Raman spectral data.

23. The automatic material supplementing method based on the real-time dynamic measurement result of any one of claims 9 to 22, wherein selecting one component or the combination of multiple components as the marker based on the historical rate of change of each component comprises:
taking one component or a combination of multiple components among N components with the highest historical rate of changes as the marker, wherein N is a positive integer.

24. A non-transitory computer-readable storage medium storing a computer program thereon, wherein the computer program, when executed by a processor, implements steps of the automatic material supplementing method based on the real-time dynamic measurement result of any one of claims 1 to 6 and claims 9 to 23.

25. A computer device comprising a memory and a processor, wherein the memory stores thereon a computer program that is able to run on the processor, and the computer program, when executed by the processor, implements steps of the automatic material supplementing method based on the real-time dynamic measurement result of any one of claims 1 to 6 and claims 9 to 23.

26. A computer program product comprising instructions which, when executed by a processor, implement the automatic material supplementing method based on the real-time dynamic measurement result of any one of claims 1 to 6 and claims 9 to 23.
